(19) Europäisches Patentamt
European Patent Office
Office européen des brevets

(11) EP 1 430 875 B1

# (12) FASCICULE DE BREVET EUROPEEN

(45) Date de publication et mention de la délivrance du brevet:
**03.10.2007 Bulletin 2007/40**

(51) Int Cl.:
*A61K 8/81* (2006.01) *A61K 8/37* (2006.01)
*A61K 8/92* (2006.01) *A61Q 5/08* (2006.01)
*A61Q 5/10* (2006.01)

(21) Numéro de dépôt: **03293211.3**

(22) Date de dépôt: **18.12.2003**

(54) **Composition anhydre sous forme de pâte pour la décoloration et la coloration simultanée des fibres kératiniques humaines**

Wasserfreie Paste zum gleichzeitigen Bleichen und Färben von menschlichen Keratinfasern

Anhydrous paste composition for simultaneous bleaching and dyeing of human keratin fibers

(84) Etats contractants désignés:
**AT BE BG CH CY CZ DE DK EE ES FI FR GB GR HU IE IT LI LU MC NL PT RO SE SI SK TR**

(30) Priorité: **20.12.2002 FR 0216404**

(43) Date de publication de la demande:
**23.06.2004 Bulletin 2004/26**

(73) Titulaire: **L'ORÉAL**
**75008 Paris (FR)**

(72) Inventeurs:
- **Legrand, Frédéric**
**92400 Courbevoie (FR)**
- **Millequant, Jean-Marie**
**94100 Saint Maur des Fosses (FR)**
- **De La Mettrie, Rolland**
**78110 Le Vesinet (FR)**

(74) Mandataire: **Casalonga, Axel**
**Bureau Casalonga & Josse**
**Bayerstrasse 71/73**
**80335 München (DE)**

(56) Documents cités:

| | |
|---|---|
| EP-A- 0 394 930 | EP-A- 0 882 444 |
| EP-A- 1 219 285 | EP-A- 1 228 751 |
| WO-A-00/10519 | DE-A- 3 814 356 |
| DE-A- 3 814 685 | US-A- 4 170 637 |

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 430 875 B1

## Description

L'invention a pour objet une composition anhydre sous forme de pâte pour la décoloration et la coloration simultanée des fibres kératiniques humaines, et en particulier des cheveux, comprenant au moins un sel peroxygéné, au moins un agent alcalin, au moins un liquide organique inerte, et au moins un colorant direct cationique, ainsi qu'un procédé de décoloration et de coloration simultanée.

Dans le domaine capillaire, on peut distinguer deux types de coloration. Le premier est la coloration permanente ou coloration d'oxydation qui utilise des colorants d'oxydation qui développent leur pouvoir tinctorial en présence d'agents oxydants. Le second est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs ; ils peuvent être mis en oeuvre avec ou sans agent oxydant. En présence d'agent oxydant, la coloration est dite éclaircissante ; sans agent oxydant, c'est la teinture directe classique non éclaircissante.

La présente invention concerne la coloration directe éclaircissante, elle est donc effectuée en présence d'un agent oxydant.

Il est connu d'avoir recours à des compositions alcalines éclaircissantes prêtes à l'emploi à base de peroxyde d'hydrogène et d'au moins un colorant direct pour éclaircir et colorer simultanément les cheveux.

De telles compositions résultent le plus souvent du mélange avant emploi d'une composition aqueuse de peroxyde d'hydrogène et d'une composition alcaline à base d'au moins un colorant direct.

Toutefois, les performances éclaircissantes de ce type de compositions restent limitées, plus particulièrement pour des applications sur des fonds foncés. On a alors recours à des compositions de décoloration à base d'au moins un sel peroxygéné (préférentiellement le persulfate) afin d'obtenir un éclaircissement plus important. Le procédé en un temps consiste à appliquer, sur la zone de chevelure que l'on souhaite traiter, une composition pulvérulente de décoloration et de coloration simultanée des cheveux prête à l'emploi contenant au moins du peroxyde d'hydrogène, au moins un sel peroxygéné (préférentiellement le persulfate), au moins un agent alcalin et au moins un colorant direct. De telles compositions prêtes à l'emploi sont notamment décrites dans les demandes de brevets DE 19 721 785 et DE 19 721 797.

De même, la demande de brevet DE 3 814 685 décrit des compositions de décoloration et de coloration simultanée des cheveux prêtes à l'emploi consistant au mélange avant emploi, d'une composition anhydre pulvérulente de décoloration contenant au moins un sel peroxygéné, au moins un agent alcalin et en outre, au moins un colorant direct; et d'une composition aqueuse de peroxyde d'hydrogène.

Ces compositions présentent l'inconvénient d'être difficile à mettre en oeuvre notamment à cause de la volatilité de la composition de décoloration pulvérulente.

En effet, les compositions décolorantes pour cheveux les plus couramment utilisées à ce jour se présentent sous forme de poudres (mélanges) de faible granulométrie, c'est à dire avec des particules dont la taille est généralement inférieure au millimètre, de préférence inférieure à quelques centaines de microns, ce qui permet une dissolution et/ou une dispersion facile et rapide dans l'eau oxygénée. Toutefois, de telles compositions pulvérulentes, compte tenu de l'état finement divisé dans lequel elles se trouvent, présentent plusieurs inconvénients : elles sont fortement volatiles et émettent ainsi, lors de leur manipulation, des poussières nocives, car les matériaux qui composent ces poudres (persulfates, silicates alcalins) sont corrosifs, irritants pour les yeux, pour les voies respiratoires et les muqueuses ; ces poudres sont par ailleurs délicates non seulement à manipuler mais également à doser (problème de poussièrage et de coulabilité). On a plus récemment développé des pâtes qui comprennent lesdits agents pulvérulents dans un support liquide organique inerte épaissi. De telles compositions sont notamment décrites dans les demandes de brevets DE-3814 356 A1, DE-197 23 538 C1 et le brevet américain N° 4 170 637.

Il existe un réel besoin de disposer de compositions pour la décoloration et la coloration simultanée, qui permettent de s'affranchir des problèmes de volatilité des compositions pulvérulentes.

Les compositions de décoloration et de coloration simultanée ne donnent généralement pas de colorations très chromatiques, de par la destruction des colorants par les sels peroxygénés très puissants. Ainsi se pose le problème de la stabilité et du stockage des colorants directs au sein de la composition.

De manière inattendue et avantageuse, la demanderesse a découvert que le problème des compositions de l'art antérieur pouvait être résolu au moyen d'une composition anhydre pâteuse pour la décoloration et la coloration simultanée des fibres kératiniques humaines, et en particulier des cheveux, comprenant dans un milieu approprié pour la teinture au moins un sel peroxygéné, au moins un agent alcalin, au moins un liquide organique inerte, et au moins un colorant direct cationique.

Cette pâte permet l'obtention d'une composition stable au stockage et permet l'obtention de couleurs plus chromatiques et plus lumineuses. Les couleurs obtenues sont vives et présentent des nuances puissantes.

L'invention porte sur une composition anhydre pâteuse pour la décoloration et la coloration simultanée des fibres kératiniques humaines, et en particulier des cheveux, au moins un sel peroxygéné, au moins un agent alcalin, un

liquide organique inerte, et au moins un colorant direct cationique.

L'invention a pour autre objet une composition prête à l'emploi destinée à la décoloration et la coloration simultanée des fibres kératiniques humaines, et en particulier des cheveux.

L'invention concerne également un procédé de décoloration et de coloration simultanée des fibres kératiniques humaines, et en particulier des cheveux, ainsi que des dispositifs à plusieurs compartiments ou "kits.

D'autres caractéristiques, aspects, objets et avantages de l'invention apparaîtront encore plus clairement à la lecture de la description et des exemples qui suivent.

L'invention a pour objet une composition anhydre pâteuse pour la décoloration et la coloration simultanée des fibres kératiniques humaines, et en particulier des cheveux, comprenant dans un milieu approprié pour la teinture :

- au moins un sel peroxygéné,
- au moins un agent alcalin,
- 15 à 35% en poids d'au moins un liquide organique inerte par rapport au poids total de la composition choisi revendication 1, et
- au moins un colorant direct cationique.

Par "composition anhydre pâteuse", on entend, au sens de l'invention, une pâte dont la teneur en eau est inférieure à 1%, de préférence inférieure à 0,5% en poids.

Par "liquide organique inerte", on entend au sens de l'invention, un liquide n'interagissant pas chimiquement avec les sels peroxygénés, ni avec les autres constituants de la composition.

Par "colorant direct cationique", on entend au sens de l'invention un colorant porteur d'au moins un atome d'azote quaternisé.

<u>Les sels peroxygénés</u> sont choisis parmi les persulfates, les perborates, les percarbonates, les peroxydes de métaux alcalins et alcalinoterreux. De préférence, on utilisera les persulfates, et plus préférentiellement les persulfates de sodium et de potassium.

Les sels peroxygénés utilisés selon l'invention représentent de préférence de 10 à 70%, et de préférence 20 à 60% en poids total de la composition.

<u>Les agents alcalins</u> sont choisis parmi l'urée, les sels d'ammonium comme le chlorure d'ammonium, le sulfate d'ammonium, le phosphate d'ammonium ou le nitrate d'ammonium, les silicates, les phosphates ou les carbonates de métaux alcalins ou alcalino-terreux, tels que le lithium, le sodium, le potassium, le magnésium, le calcium, le baryum.

Les agents alcalins utilisés selon l'invention représentent de préférence de 0,01 à 40%, et de préférence 0,1 à 30% en poids total de la composition.

<u>Le liquide organique inerte</u>

Selon l'invention, il peut être choisi dans le groupe formé par les polydécènes de formule $C_{10n}\,H_{[(20n)+2]}$ avec n variant de 3 à 9, les esters d'alcools gras ou d'acides gras, les esters ou di-esters de sucres d'acides gras en $C_{12}$-$C_{24}$, les éthers cycliques ou les esters cycliques, les huiles de silicone, les huiles minérales ou les huiles végétales.

<u>Polydécènes de formule $C_{10n}H_{[(20n)+21]}$ avec n variant de 3 à 9.</u>

Ces composés répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes. Parmi ces composés, on choisit plus particulièrement selon l'invention ceux pour lesquels dans la formule, n varie de 3 à 7.

On peut citer, à titre d'exemple, et de préférence, le produit vendu sous la dénomination SILKFLO® 366 NF POLYDECENE par la société AMOCO CHEMICAL, ceux vendus sous la dénomination NEXBASE® 2002 FG, 2004 FG, 2006 FG et 2008 FG par la société FORTUM.

Dans la composition anhydre de décoloration et de coloration selon l'invention, le ou les polydécènes sont présents de préférence dans une proportion pondérale allant d'environ 15 à 35% et plus particulièrement d'environ 15 à 25% par rapport au poids total de la pâte.

<u>Esters d'alcools gras ou d'acides gras.</u>

Parmi les esters d'alcools gras ou d'acides gras, on entend notamment :

- les esters de monoalcools inférieurs saturés linéaires ou ramifiés en $C_3$-$C_6$, avec des acides gras monofonctionnels en $C_{12}$-$C_{24}$ ( ces derniers pouvant être linéaires ou ramifiés, saturés ou insaturés et choisis notamment parmi les

oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates, etc...). Parmi lesdits esters, on préfère plus particulièrement utiliser le palmitate d'isopropyle, le myristate d'isopropyle,

- les esters de monoalcools linéaires ou ramifiés en $C_3$-$C_8$, avec des acides gras bifonctionnels en $C_8$-$C_{24}$ ( ces derniers pouvant être linéaires ou ramifiés, saturés ou insaturés), comme par exemple le di-ester isopropylique de l'acide sébacique (sébaçate de di-isopropyle),
- les esters de monoalcools linéaires ou ramifiés en $C_3$-$C_8$, avec des acides gras bifonctionnels en $C_2$-$C_8$ ( ces derniers pouvant être linéaires ou ramifiés, saturés ou insaturés), comme par exemple l'adipate de di-octyle et le maléate de di-caprylyle,
- l'ester d'un acide trifonctionnnel comme le citrate de tri-éthyle.

Esters et di-esters de sucres d'acides gras en $C_{12}C_{24}$.

Par "sucre", on entend des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en $C_{12}$-$C_{24}$, linéaires ou ramifiés, saturés ou insaturés.

Les esters peuvent être choisis parmi les les mono-, di-, tri- et tétra-esters, les polyesters et leurs mélanges.

Lesdits esters peuvent être notamment choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates, etc...

Plus particulièrement selon l'invention, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer, à titre d'exemple, et de préférence, le produit vendu sous la dénomination GLUCATE DO par la société AMERCHOL, qui est un dioléate de méthylglucose.

On peut aussi citer à titre d'exemples d'esters ou de mélanges d'esters de sucre d'acide gras:

- les produits vendus sous les dénominations F160, F140, F110, F90, F70, SL40 par la société CRODESTA, désignant respectivement les palmito-stéarates de sucrose formés de 73% de monoester et 27% de di-et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le monolaurate de sucrose;
- les produits vendus sous la dénomination RYOTO SUGAR ESTERS par exemple référencés B370 et correspondant au béhénate de saccharose formé de 20% de monoester et 80% de di-triester-polyester;
- le mono-di-palmito-stéarate de sucrose commercialisé par la société GOLDSCHMIDT sous la dénomination TEGOSOFT PSE.

Ethers cycliques et esters cycliques

Selon l'invention, ils peuvent notamment être choisis parmi la γ-butyrolactone, le diméthyl isosorbide, ou le diisopropyl isosorbide.

Huiles de silicone

Les huiles de silicone sont des fluides de silicones liquides et non volatiles de viscosité inférieure ou égale à 10 000 mPa.s à 25°C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment par exemple les huiles de silicones vendues sous les dénominations DC-200 fluid- 5 mPa.s, DC-200 fluid- 20 mPa.s, DC-200 fluid- 350 mPa.s, DC-200 fluid- 1000 mPa.s, DC-200 fluid- 10 000 mPa.s par la société DOW CORNING.

Huiles minérales

Parmi les huiles minérales, on peut citer notamment l'huile de paraffine.

Huiles végétales

Parmi les huiles végétales, on peut citer notamment l'huile d'avocat, l'huile d'olive ou la cire liquide de jojoba.
De préférence selon l'invention, le liquide organique inerte est choisi parmi les polydécènes et les esters d'alcools gras ou d'acides gras.
Dans les pâtes selon l'invention, le liquide organique inerte est de préférence présent à une concentration allant d'environ 15 à 35% en poids par rapport au poids total de la pâte.
La demanderesse a également observé que des compositions préférées permettant l'obtention des couleurs les plus stables sont obtenues en utilisant de préférence, des colorants directs cationiques choisis parmi les colorants xanthèniques, azoïques, azométhiniques et méthiniques.
De préférence, on utilisera des colorants directs cationiques hétérocycliques, et plus particulièrement ce colorant possède au moins une charge cationique sur un hétérocycle.
Encore plus préférentiellement, on utilisera des colorants directs porteurs d'au moins une charge cationique sur un hétérocycle.
Tout particulièrement on utilisera des colorants azoïques, méthiniques ou azométhiniques porteurs d'au moins une charge cationique sur un hétérocycle.
Selon l'invention, le colorant direct cationique est choisi parmi les colorants suivants :

- les colorants xanthèniques cationiques, parmi lesquels on utilise de préférence l'Acid Red 52,
- les colorants directs azoïques ou azométhiniques cationiques, parmi lesquels on peut utiliser de préférence le Basic Blue 41, le Basic Blue 67, le Basic Brown 1, le Basic Brown 4, le Basic Red 18, le Basic Red 22, le Basic Red 46, le Basic Red 104, le Basic Violet 35, le Basic Yellow 45, le Basic Yellow 57 et le Basic Yellow 67,
- les colorants directs méthiniques cationiques, comme notamment le Basic Red 14, le Basic Yellow 13 et le Basic Yellow 29

ainsi que les colorants décrits dans la demande de brevet EP 1 025 834 suivants :

- De formule (I)

$$G\text{-}N{=}N\text{-}J \qquad (I)$$

dans laquelle
le symbole G représente un groupement choisi parmi les structures $G_1$ à $G_3$ suivantes :

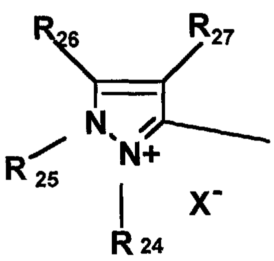

$G_1$

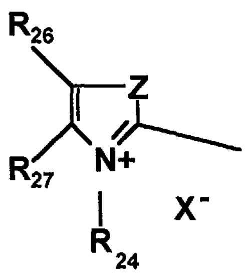

$G_2$

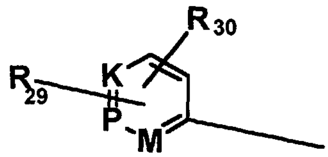

$G_3$

dans lesquelles,

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;

$R_{25}$ désigne un radical alkyle en $C_1$-$C_4$ ou un radical phényle;

$R_{26}$ et $R_{27}$, identiques ou différents, représentent un radical alkyle en $C_1$-$C_4$ , un radical phényle, ou forment ensemble dans $G_1$ un cycle benzénique substitué par un ou plusieurs radicaux alkyle en $C_1$-$C_4$ , alcoxy en $C_1$-$C_4$, ou $NO_2$ ou forment ensemble dans $G_2$ un cycle benzénique éventuellement substitué par un ou plusieurs radicaux alkyle en $C_1$-$C_4$ , alcoxy en $C_1$-$C_4$, ou $NO_2$;

$R_{26}$ peut désigner en outre un atome d'hydrogène;

Z désigne un atome d'oxygène, de soufre ou un groupement $-NR_{25}$;

M représente un groupement -CH, -CR (R désignant alkyle en $C_1$-$C_4$), ou -NR28$(X^-)_r$;

K représente un groupement -CH, -CR (R désignant alkyle en $C_1$-$C_4$), ou $-NR_{28}(X^-)_r$;

P représente un groupement -CH, -CR (R désignant alkyle en $C_1$-$C_4$), ou $-NR_{28}(X^-)_r$;

r désigne zéro ou 1;

$R_{28}$ représente un atome O-, un radical alcoxy en $C_1$-$C_4$, ou un radical alkyle en $C_1$-$C_4$;

$R_{29}$ et $R_{30}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$ , un radical $-NO_2$;

$X^-$ représente un anion de préférence choisi parmi le chlorure, l'iodure, le méthyl sulfate, l'éthyl sulfate, l'acétate et le perchlorate;

le symbole J représente :

- (a) un groupement de structure $J_1$ suivante :

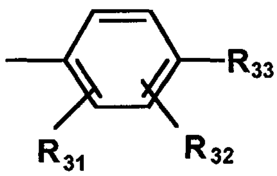

$J_1$

dans laquelle,

$R_{31}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{34}$, $-NR_{35}R_{36}$, -NHCOalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre;

$R_{32}$ représente un atome d'hydrogène, un atome d'halogène choisi parmi le chlore, le brome, l'iode et le fluor, un radical alkyle en $C_1$-$C_4$, alcoxy en $C_1$-$C_4$ ou forme avec $R_{33}$ ou $R_{34}$ un cycle à 5 ou 6 chaînons contenant ou non un ou plusieurs hétéroatomes choisis parmi l'azote, l'oxygène ou le soufre;

$R_{33}$ représente un atome d'hydrogène, un radical -OH, un radical $-NHR_{34}$, un radical $-NR_{35}R_{36}$; $R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;

$R_{35}$ et $R_{36}$, identiques ou différents, représentent un radical alkyle en $C_1$-$C_4$ , un radical monohydroxyalkyle en $C_1$-$C_4$, polyhydroxyalkyle en $C_2$-$C_4$;

- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle,
  et notamment un groupement de structure $J_2$ suivante :

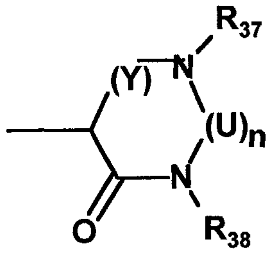

$J_2$

dans laquelle,
$R_{37}$ et $R_{38}$, identiques ou différents, représentent un atome d'hydrogène , un radical alkyle en $C_3$-$C_{10}$, un radical phényle;
Y désigne le radical -CO- ou le radical -C($CH_3$)= ;
n représente 0 ou 1, avec, lorsque n désigne 1, U désigne le radical -CO-

- de formule (II) suivante :

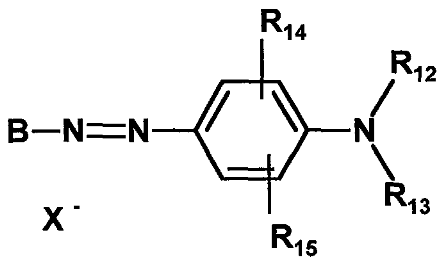

(II)

dans laquelle :

$R_{12}$ représente un atome d'hydrogène ou un radical alkyle en $C_1$-$C_4$,
$R_{13}$ représente un atome d'hydrogène, un radical alkyle pouvant être substitué par un radical -CN ou par un groupement amino, un radical 4'-aminophényle ou forme avec $R_{12}$ un hétérocycle éventuellement oxygéné et/ou azoté pouvant être substitué par un radical alkyle en $C_1$-$C_4$,
$R_{14}$ et $R_{15}$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,
$X^-$ représente un anion de préférence choisi parmi le chlorure, le méthyl sulfate et l'acétate,
B représente un groupement choisi par les structures B1 à B6 suivantes :

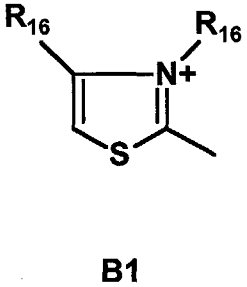

;

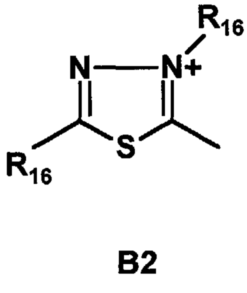

;

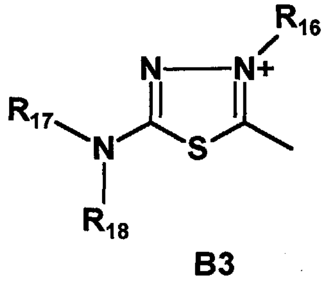

;

B1 B2 B3

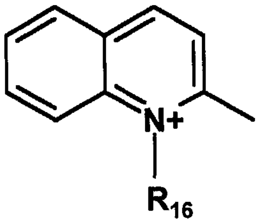 ; 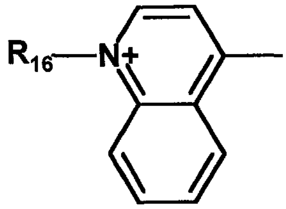 et 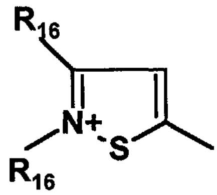 ;

B4 B5 B6

dans lesquelles $R_{16}$ représente un radical alkyle en $C_1$-$C_4$,
$R_{17}$ et $R_{18}$, identiques ou différents, représentent un atome d'hydrogène ou un radical alkyle en $C_1$-$C_4$

- les composés de formules (III) et (IV) suivantes :

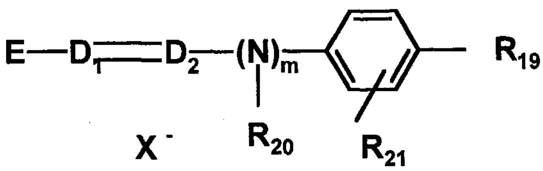

(III)

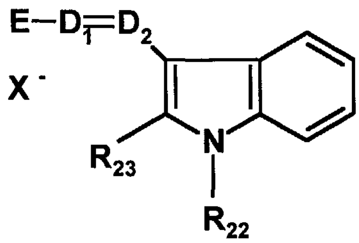

(IV)

dans lesquelles :

$R_{19}$ représente un atome d'hydrogène, un radical alcoxy en $C_1$-$C_4$, un atome d'halogène tel que le brome, le chlore, l'iode ou le fluor ou un radical amino,
$R_{20}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{21}$ représente un atome d'hydrogène ou d'halogène tel que le brome, le chlore, l'iode ou le fluor,
$R_{22}$ et $R_{23}$, identiques ou différents, représentent un atome d'hydrogène ou un radical alkyle en $C_1$-$C_4$,
$D_1$ et $D_2$, identiques ou différents, représentent un atome d'azote ou le groupement -CH,
m représente 0 ou 1,
étant entendu que lorsque $R_{19}$ représente un groupement amino non substitué, alors D, et $D_2$ représentent simultanément un groupement -CH et m = 0,
$X^-$ représente un anion de préférence choisi parmi le chlorure, le méthyl sulfate et l'acétate,
E représente un groupement choisi par les structures E1 à E8 suivantes :

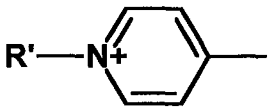

E1

;

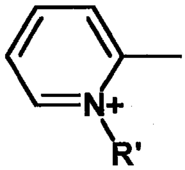

E2

;

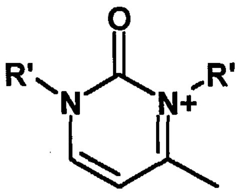

E3

;

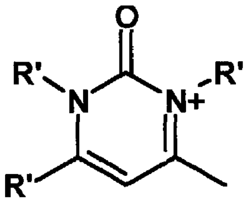

E4

;

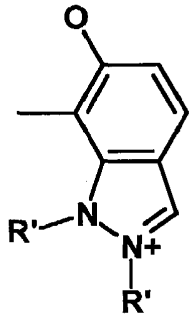

E5

;

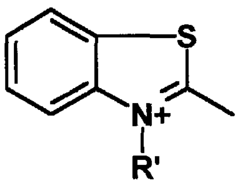

E6

;

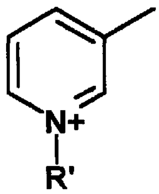

E7

et

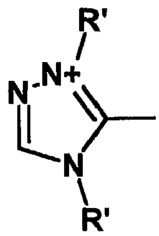

E8

dans lesquelles R' représente un radical alkyle en $C_1$-$C_4$ ;
lorsque m représente 0 et que $D_1$ représente un atome d'azote, alors E peut également désigner un groupement de structure E9 suivante :

E9

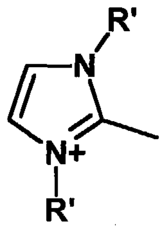

dans laquelle R' représente un radical alkyle en $C_1$-$C_4$.

- les composés de formule (V) suivante :

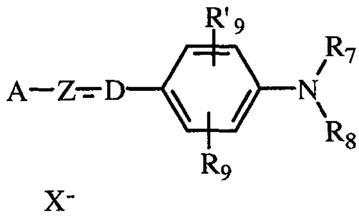

(V)

dans laquelle :

Z et D représentent, identiques ou différents, un atome d'azote ou le groupement -CH,
$R_7$ et $R_8$, identiques ou différents, représentent un atome d'hydrogène ; un radical alkyle en $C_1$-$C_4$ pouvant être substitué par un radical -CN, -OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,
$R_9$ et $R'_9$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,
$X^-$ représente un anion de préférence choisi parmi le chlorure, le méthyl sulfate et l'acétate,
A représente un groupement choisi par les structures A1 à A19 suivantes :

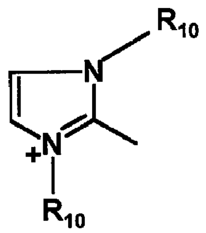

$A_1$ ;

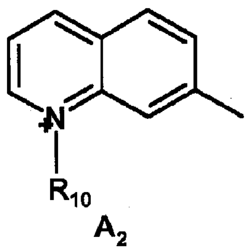

$A_2$ ;

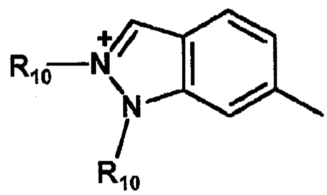

$A_3$ ;

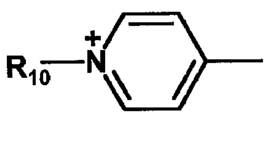

$A_4$ ;

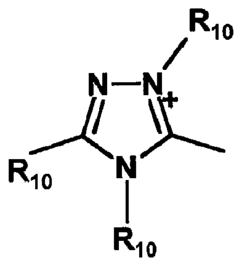

$A_5$ ;

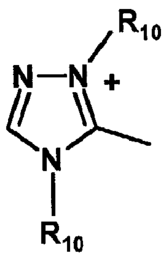

$A_6$ ;

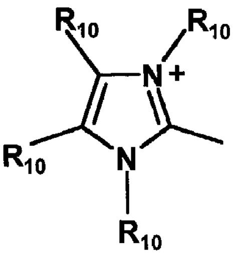

$A_7$ ;

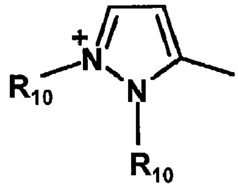

$A_8$ ;

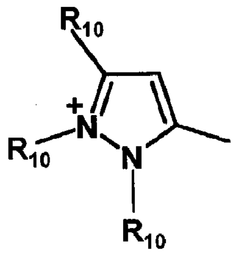

$A_9$ ;

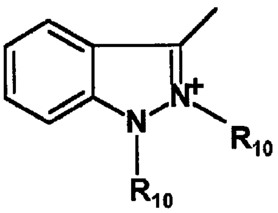

$A_{10}$ ;

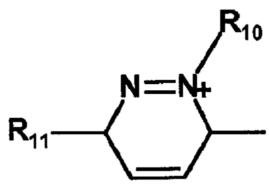

$A_{11}$ ;

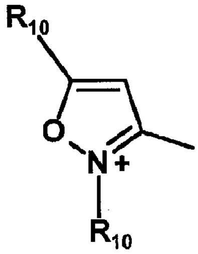

$A_{12}$ ;

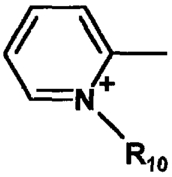

$A_{13}$ ;

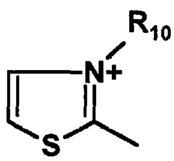

$A_{14}$ ;

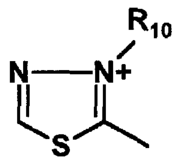

$A_{15}$ ;

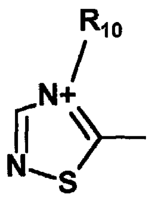

$A_{16}$ ;

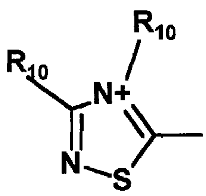

$A_{17}$ ;

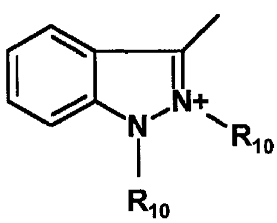

$A_{18}$

et

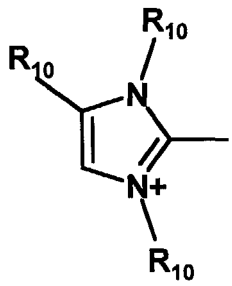

$A_{19}$

dans lesquelles $R_{10}$ représente un radical alkyle en $C_1$-$C_4$ pouvant être substitué par un radical hydroxyle et $R_{11}$ représente un radical alcoxy en $C_1$-$C_4$,

- les colorants décrits dans la demande de brevet EP 714954 de formules :

**(VI)**

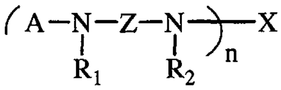

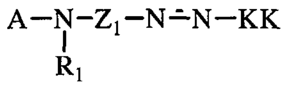

**(VII)**

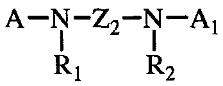

**(VIII)**

dans lesquelles
A et $A_1$, indépendamment l'un de l'autre, sont les restes de formule :

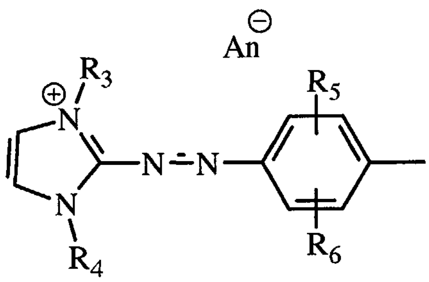

Z représente un reste de diamine aliphatique ou aromatique,
$R_1$ et $R_2$, indépendamment l'un de l'autre, représentent un atome d'hydrogène, un groupe alkyle en $C_1$-$C_4$ ou peuvent former ensemble avec 2 atomes d'azote auxquels ils sont rattachés ou avec Z et $Z_2$ un cycle à 5, 6 ou 7 chaînons,
X représente le reste d'un chaînon formant un pont,
n représente un nombre entier 2, 3 ou 4,
$Z_1$ représente un reste de diamines aromatique,
$Z_2$ représente un reste de diamines aliphatique,
KK représente un reste de composé coupleur,
$R_3$ et $R_4$, indépendamment l'un de l'autre, représentent un atome d'hydrogène ou un groupe alkyle en $C_1$-$C_4$,
$R_5$ et $R_6$, indépendamment l'un de l'autre, représentent un atome d'hydrogène, un groupe alkyle en $C_1$-$C_4$ ou

un groupe alkoxy en $C_1$-$C_4$,
An représente un anion incolore,

ainsi que les colorants cationiques décrits dans les demandes de brevet WO 95/01 772, WO 95/15 144, EP 714 954, EP 1 170 000 EP 1 166 753, EP 1 166 754 et EP 1 170 001, différents des colorants ci-dessus. Le passage de ces demandes consacré aux colorants cationiques est incorporé dans la présente demande.

De manière préférée, on utilisera les colorants Basic Red 51 de formule (IX) :

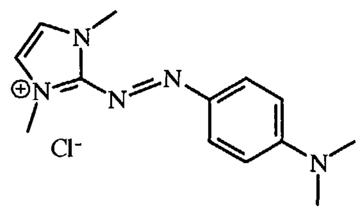

**(IX)**

le Basic Yellow 87 de formule (X) :

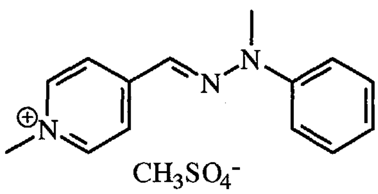

**(X)**

ainsi que le Basic Orange 31 de formule (XI) :

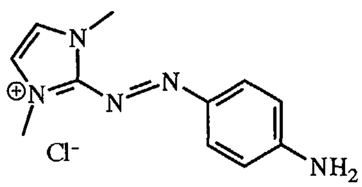

**(XI)**

Le ou les colorants directs cationiques utilisés selon l'invention représentent de préférence de 0,001 à 20%, et de préférence 0,01 à 10%, et plus préférentiellement de 0,1 à 5% en poids total de la composition.
Ces compositions peuvent contenir au moins un polymère amphiphile de préférence non ionique et/ou anionique comportant au moins une chaîne grasse. Classiquement, ces épaississants traditionnels engendrant une chute de la viscosité des compositions décolorantes au cours du temps, la Demanderesse a plus récemment proposé, dans le brevet français 2 788 974, d'utiliser un système épaississant capable d'assurer le maintien d'une viscosité élevée pendant la durée nécessaire pour obtenir la décoloration souhaitée, et consistant à associer l'épaississant hydrosoluble classique à un polymère amphiphile non ionique comportant au moins une chaîne grasse.
Par ailleurs, comme les traitements de décoloration est souvent agressif et conduit à de mauvaises propriétés cosmétiques des cheveux tels qu'un démêlage difficile, un toucher désagréable ou des cheveux rêches et ternes, et surtout à une dégradation des fibres, la Demanderesse a proposé dans le brevet français N° 2 788 976 de limiter significativement cette dégradation en employant une association de polymères amphiphiles non ioniques et/ou anioniques et de polymères substantifs cationiques ou amphotères.

Polymères amphiphiles non ioniques comportant au moins une chaîne grasse

Ils sont choisis de préférence parmi :

- (1) les celluloses modifiées par des groupements comportant au moins une chaîne grasse ; on peut citer à titre d'exemple :

  - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS® (alkyles en $C_{16}$) vendu par la société AQUALON, ou le produit BERMOCOLL EHM 100® vendu par la société BEROL NOBEL,
  - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.

- (2) les hydroxypropylguars modifiés par des groupements comportant au moins une chaîne grasse en $C_8$ à $C_{22}$, tel que le produit JAGUAR® XC-95/3 (chaîne alkyle en $C_{14}$) vendu par la société RHODIA, le produit ESAFLOR® HM 22 (chaîne alkyle en $C_{22}$) vendu par la société LAMBERTI, les produits RE210-18® (chaîne alkyle en $C_{14}$) et RE205-1® (chaîne alkyle en $C_{20}$) vendus par la société RHONE POULENC.

- (3) les copolymères de vinyl pyrrolidone et de monomères hydrophobes à chaîne grasse dont on peut citer à titre d'exemple :

  - les produits ANTARON® V216 ou GANEX® V216 (copolymère vinylpyrrolidone / hexadécène) vendu par la société I.S.P.
  - les produits ANTARON® V220 ou GANEX® V220 (copolymère vinylpyrrolidone / eicosène) vendu par la société I.S.P.

- (4) les copolymères de méthacrylates ou d'acrylates d'alkyles en $C_1$-$C_6$ et de monomères amphiphiles comportant au moins une chaîne grasse.

- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- (6) les polymères à squelette aminoplaste éther possédant au moins une chaîne grasse, tels que les composés PURE THIX® proposés par la société SUD-CHEMIE.

- (7) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non-ioniques comportent une liaison uréthane entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut citer le Ser-ad FX 1100® de la société SERVO DELDEN qui est un copolymère dénommé sous la désignation I.N.C.I. européenne et américaine "Steareth-100/PEG-136/H.M.D.I. Copolymer".

On peut aussi utiliser le Rhéolate® 205 à fonction urée vendu par la société RHEOX ou encore les Rhéolates® 208 , 204 ou 212, ainsi que l'Acrysol@ RM 184.

On peut également citer le produit ELFACOS® T210 à chaîne alkyle en $C_{12-14}$ et le produit ELFACOS® T212

à chaîne alkyle en $C_{18}$ de chez AKZO.

Les polyéthers polyuréthanes utilisables selon l'invention sont en particulier ceux décrits dans l'article de G. Fonnum, J. Bakke et Fk. Hansen - Colloid Polym. Sci 271, 380-389 (1993).

On préfère en particulier les polyéthers polyuréthanes comportant au moins une chaîne grasse en $C_{10}$ à $C_{20}$, et les hydroxypropylguars modifiés par des groupements comportant au moins une chaîne grasse en $C_8$ à $C_{22}$.

Polymères amphiphiles anioniques comportant au moins une chaîne grasse

Les polymères amphiphiles anioniques comportant au moins une chaîne grasse utilisables selon la présente invention sont des polymères réticulés ou non réticulés, comprenant :

- des motifs hydrophiles dérivés d'un ou de plusieurs monomères à insaturation éthylénique portant une fonction acide carboxylique libre, ou une fonction sulfonique libre ou partiellement ou totalement neutralisée, et
- des motifs hydrophobes dérivés d'un ou de plusieurs monomères à insaturation éthylénique portant une chaîne latérale hydrophobe, et éventuellement
- des motifs de réticulation dérivés d'un ou plusieurs monomères polyinsaturés.

- (I) Le ou les monomères à insaturation éthylénique portant une fonction acide carboxylique sont choisis parmi l'acide éthacrylique, l'acide méthacrylique et l'acide acrylique, de préférence parmi l'acide méthacrylique, l'acide acrylique et des mélanges de ceux-ci.

Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.

(i) Les esters d'acides carboxyliques insaturés et d'alcools gras sont choisis par exemple parmi les éthacrylates, méthacrylates et/ou acrylates d'alkyles en $C_{10}$-$C_{30}$, de préférence en $C_{12}$-$C_{22}$. Ils englobent par exemple l'acrylate de lauryle, l'acrylate de stéaryle, l'acrylate de décyle, l'acrylate d'isodécyle, l'acrylate de dodécyle, et les méthacrylates correspondants, à savoir le méthacrylate de lauryle, le méthacrylate de stéaryle, le méthacrylate de décyle, le méthacrylate d'isodécyle, et le méthacrylate de dodécyle.

(ii) Les éthers allyliques d'alcools gras formant les motifs hydrophobes des polymères amphiphiles anioniques de la présente invention correspondent à la formule (1) suivante :

$$CH_2 = C\ R'\ CH_2\ O\ B_n\ R \qquad (1)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comportant de 8 à 30 atomes de carbone, de préférence 10 à 24, et plus particulièrement encore de 12 à 18 atomes de carbone. Un motif de formule (1) plus particulièrement préféré est un motif dans lequel R' désigne H, n est égal à 10, et R désigne un radical stéaryle($C_{18}$).

Ledit monomère réticulant est un composé comportant au moins deux doubles liaisons polymérisables non conjuguées l'une avec l'autre. On peut citer à tire d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

Des polymères amphiphiles anioniques du type décrit ci-dessus sont décrits et préparés, par exemple dans les brevets US-3 915 921 et US-4 509 949 (copolymères d'acide (méth)acrylique et de (méth)acrylates d'alkyles en $C_{10}$-$C_{30}$, ou dans le brevet EP-0 216 479 B2 (copolymères d'acide (méth)acrylique et d'éthers allyliques d'alcools gras).

On peut citer à titre d'exemples de polymères préférés :

- les polymères réticulés d'acide acrylique et de méthacrylate d'alkyle en $C_{10}$-$C_{30}$, tels que le CARBOPOL® ETD-2020 commercialisé par la société GOODRICH;
- les polymères réticulés d'acide acrylique et d'acrylate d'alkyle en $C_{10}$-$C_{30}$, tels que les polymères commercialisés sous les dénominations CARBOPOL® 1382, PEMULEN® TR1, PEMULEN® TR2 par la société GOODRICH;
- le terpolymère acide méthacrylique/acrylate d'éthyle/méthacrylate de stéaryle oxyéthyléné (55/35/10);
- le terpolymère acide (méth)acrylique/acrylate d'éthyle/méthacrylate de béhényle oxyéthyléné 25 OE, et
- le terpolymère réticulé acide méthacrylique/acrylate d'éthyle/stéareth-10 allyl éther.

- (II) les polymères amphiphiles comportant comme motifs hydrophiles au moins un monomère à insaturation éthy-

lénique à groupement sulfonique, sous forme libre ou partiellement ou totalement neutralisée et au moins une partie hydrophobe sont par exemple décrits dans les demandes de brevets français de la demanderesse N°-0016954 et 0100328 dont le contenu fait partie intégrante de la présente invention.
On peut notamment citer plus particulièrement parmi eux :

le copolymère acide acrylamido-2-méthyl-2-propane-sulfonique(AMPS)/n-dodécylacrylamide neutralisé par la soude, le copolymère réticulé par du méthylène-bis-acrylamide constitué de 75% en poids de motifs AMPS neutralisés par $NH_3$ et de 25% en poids de motifs acrylate de GENAPOL® T-250, le copolymère réticulé par du méthacrylate d'allyle constitué de 90% en poids de motifs AMPS neutralisés par $NH_3$ et de 10% en poids de motifs méthacrylate de GENAPOL® T-250, ou le copolymère réticulé par du méthacrylate d'allyle constitué de 80% en poids de motifs AMPS neutralisés par $NH_3$ et de 20% en poids de motifs méthacrylate de GENAPOL® T-250.

Dans la composition anhydre pâteuse pour la décoloration et la coloration simultanée selon l'invention, le ou les polymères amphiphiles non ioniques et/ou anioniques comportant au moins une chaîne grasse peuvent être présents dans une proportion pondérale allant d'environ 0,01 à 30% et de préférence d'environ 0,01 à 15% par rapport au poids total de la poudre décolorante.
La composition anhydre pâteuse selon l'invention peut contenir des polymères conditionneurs cationiques ou amphotères anhydres bien connus de l'homme de l'art et décrits dans les brevets français N°-2788974 et 2788976 et tels que décrits ci-après.

Polymères cationiques

Au sens de la présente invention, l'expression "polymère cationique" désigne tout polymère contenant des groupements cationiques et/ou des groupements ionisables en groupements cationiques.
Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.
Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.
Les polymères cationiques utilisés ont généralement une masse moléculaire moyenne en nombre comprise entre 500 et $5.10^6$ environ, et de préférence comprise entre $10^3$ et $3.10^6$ environ.
Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :

(1) Les homopolymères ou copolymères dérivés d'esters ou d'amides acryliques ou méthacryliques et comportant au moins un des motifs de formules (42), (43), (44) ou (45) suivantes:

(42) 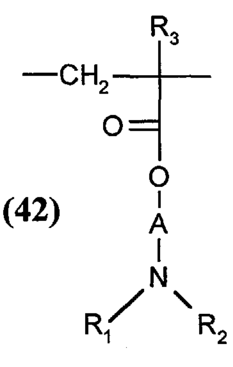

(43) 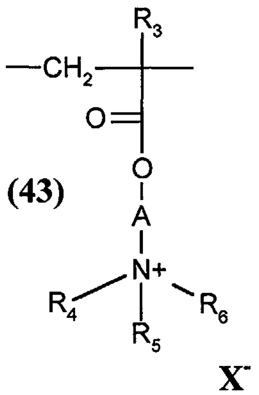

(44) 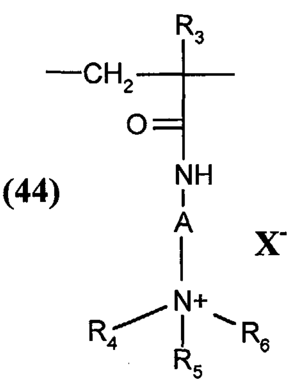

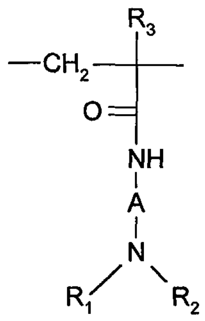

**(45)**

dans lesquelles:

$R_3$ désigne un atome d'hydrogène ou un radical $CH_3$;
A représente un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 6 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :

- les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
- les copolymères d'acrylamide et de chlorure de méthacryloyloxyéthyl-triméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxy-éthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthyl-aminopropyle quaternisés tel que le produit vendu sous la dénomination "GAFQUAT HS 100" par la société ISP.

(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .

(3) Les dérivés de cellulose cationiques tels que les copolymères de cellulose ou les dérivés de cellulose greffés

avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) Les cyclopolymères d'alkyl diallyl amine ou de dialkyl diallyl ammonium tels que les homopolymères ou copolymères comportant comme constituant principal de la chaîne des motifs répondant aux formules (46) ou (47) :

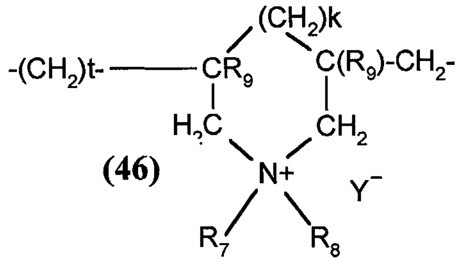

(46)

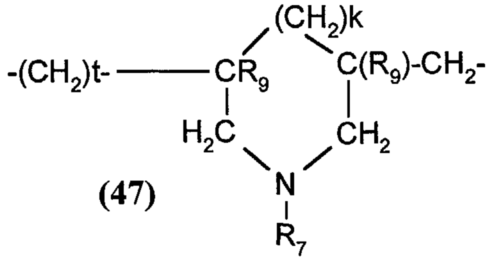

(47)

formules dans lesquelles
k et t sont égaux à 0 ou 1, la somme k + t étant égale à 1 ;
$R_9$ désigne un atome d'hydrogène ou un radical méthyle ;
$R_7$ et $R_8$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ;
$Y^-$ est un anion tel que bromure, chlorure, acétate, borate, citrate, tartrate, bisulfate, bisulfite, sulfate, phosphate.
Ces polymères sont notamment décrits dans le brevet français 2.080.759 et dans son certificat d'addition 2.190.406.
Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

(10) Le polymère de diammonium quaternaire contenant des motifs récurrents répondant à la formule :

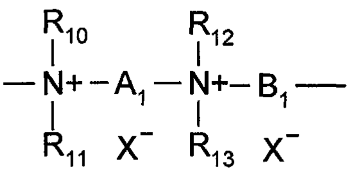

(48)

formule (48) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R10, R11, R12 et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH- $R_{14}$-D où $R_{14}$ est un alkylène et D un groupement ammonium quaternaire ;
$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements

sulfoxyde, sulfone, disulfure, amino, alkylamino, hydroxyle, ammonium quaternaire, uréido, amide ou ester, et $X^-$ désigne un anion dérivé d'un acide minéral ou organique;

$A_1$, $R_{10}$ et $R_{12}$ peuvent former avec les deux atomes d'azote auxquels ils sont rattachés un cycle pipérazinique ; en outre si $A_1$ désigne un radical alkylène ou hydroxyalkylène linéaire ou ramifié, saturé ou insaturé, $B_1$ peut également désigner un groupement $-(CH_2)n-CO-D-OC-(CH_2)n-$

dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

ù x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;

c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- .

De préférence, $X^-$ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (49) suivante:

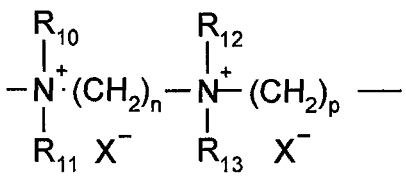

**(49)**

dans laquelle $R_{10}$, $R_{11}$, $R_{12}$ et $R_{13}$, identiques ou différents, désignent un radical alkyle ou hydroxyalkyle ayant de 1 à 4 atomes de carbone environ,

n et p sont des nombres entiers variant de 2 à 20 environ et,

X- est un anion dérivé d'un acide minéral ou organique.

(11) Les polymères de polyammonium quaternaire constitués de motifs de formule (50) :

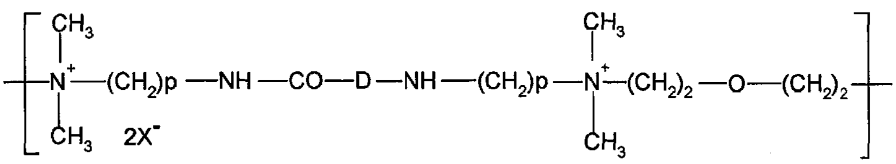

**(50)**

dans laquelle :

p désigne un nombre entier variant de 1 à 6 environ,
D peut être nul ou peut représenter un groupement $—(CH_2)_r—CO—$ dans lequel r désigne un nombre égal à 4 ou à 7, et
$X^-$ est un anion dérivé d'un acide minéral ou organique.

Les polymères cationiques comportant des motifs de formule (50) sont notamment décrits dans la demande de brevet EP-A-122 324 et peuvent être préparés selon les procédés décrits dans les brevets U.S.A. n° 4 157 388, 4 390 689, 4 702 906, 4 719 282.
Parmi ces polymères, on préfère ceux de masse moléculaire mesurée par RMN du Carbone 13 inférieure à 100000, et dans la formule de laquelle :

p est égal à 3, et,

a) D représente un groupement $—(CH_2)_4—CO—$ , X désigne un atome de chlore, la masse moléculaire mesurée par RMN du Carbone 13 ( $RMN^{13}C$ ) étant d'environ 5600 ; un polymère de ce type est proposé par la société MIRANOL sous le nom de MIRAPOL-AD1,
b) D représente un groupement $-(CH_2)_7—CO—$ , X désigne un atome de chlore, la masse moléculaire mesurée par RMN du Carbone 13 ( $RMN^{13}C$ ) étant d'environ 8100 ; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, ( $RMN^{13}C$ ) étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9, (masse moléculaire $RMN^{13}C$, environ 7800) MIRAPOL-175, (masse moléculaire $RMN^{13}C$, environ 8000) MIRAPOL-95, (masse moléculaire $RMN^{13}C$, environ 12500).

Plus particulièrement encore, on préfère selon l'invention le polymère à motifs de formule (50) dans laquelle p est égal à 3, D désigne la valeur zéro, X désigne un atome de chlore, la masse moléculaire mesurée par RMN du Carbone 13, ( $RMN^{13}C$ ) étant d'environ 25500.

(12) Les polymères quaternaires de vinylpyrrolidone et de vinylimidazole tels que par exemple les produits commercialisés sous les dénominations Luviquat FC 905, FC 550 et FC 370 par la société B.A.S.F.

(13) Les polyamines comme le Polyquart H vendu par HENKEL, référencé sous le nom de " POLYETHYLENEGLYCOL (15) TALLOW POLYAMINE " dans le dictionnaire CTFA.

(14) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes :

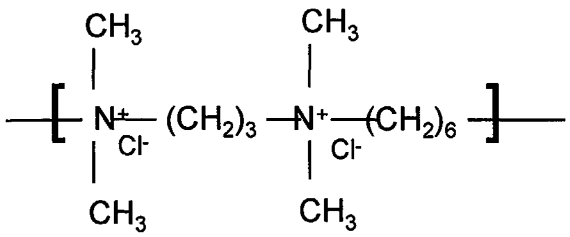

(W)

et notamment ceux dont le poids moléculaire, déterminé par chromatographie par perméation de gel, est compris entre 9500 et 9900;

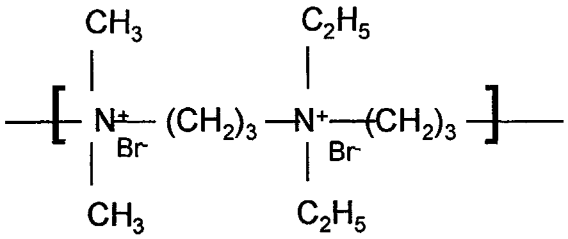

(U)

et notamment ceux dont le poids moléculaire, déterminé par chromatographie par perméation de gel, est d'environ 1200.

<u>Polymères amphotères</u>

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.

Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 6 atomes de carbone et plus particulièrement le N-éthylacrylamide, le N-tertiobutyl-acrylamide, ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'amino-éthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethyl méthacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$\left[CO—R_{19}—CO—Z\right] \qquad \textbf{(51)}$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

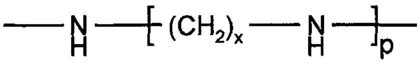

**(52)**

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (52) ci-dessus, dans lequel x=2 et p=1 et qui dérive de l'éthylènediamine, ou le radical dérivant de la pipérazine :

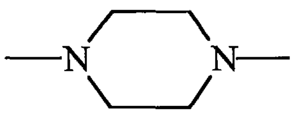

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels. Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule :

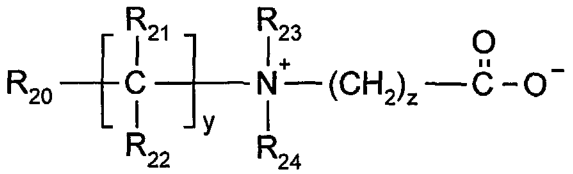

**(53)**

dans laquelle $R_{20}$ désigne un groupement insaturé polymérisable tel qu'un groupement acrylate, méthacrylate, acrylamide ou méthacrylamide, y et z représentent un nombre entier de 1 à 3,
$R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane décrits notamment dans les brevets français N°-2137684 ou US-3879376, comportant des motifs monomères répondant aux formules (54), (55), (56) suivantes réunies dans leur chaîne:

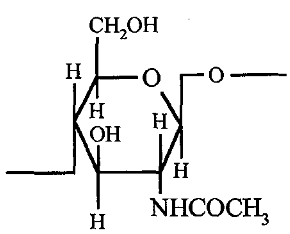

**(54)**

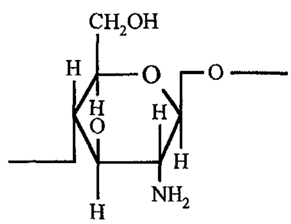

**(55)**

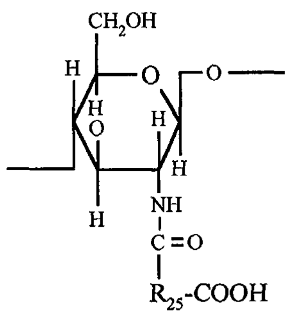

**(56)**

le motif (54) étant présent dans des proportions comprises entre 0 et 30%, le motif (55) dans des proportions comprises entre 5 et 50% et le motif (56) dans des proportions comprises entre 30 et 90%, étant entendu que dans ce motif (56), $R_{25}$ représente un radical de formule :

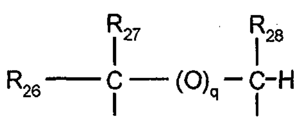

dans laquelle q désigne zéro ou 1 ;
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.
Des polymères de ce type plus particulièrement préférés comportent de 0 à 20% en poids de motifs (54), de 40 à

50% en poids de motifs (55), et de 40 à 50% en poids de motifs (56) dans lequel $R_{25}$ désigne le radical $-CH_2-CH_2-$ ;

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (52) tels que ceux décrits par exemple dans le brevet français 1 400 366 :

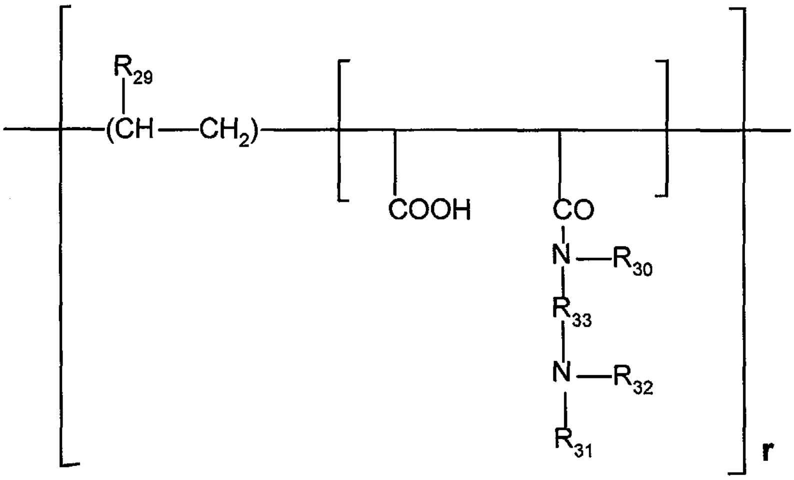

(57)

dans laquelle $R_{29}$ représente un atome d'hydrogène, un radical $CH_3O$ $CH_3CH_2O$ phényle, $R_{30}$ désigne l'hydrogène ou un radical alkyle inférieur tel que méthyle, éthyle, $R_{31}$désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$ , $-CH_2-CH_2-CH_2-$ , $-CH_2-CH(CH_3)-$ , $R_{31}$ ayant les significations mentionnées ci-dessus, ainsi que les homologues supérieurs de ces radicaux et contenant jusqu'à 6 atomes de carbone, r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

- D-X-D-X-D- (58)

où D désigne un radical

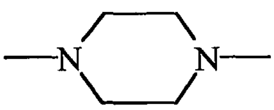

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

- D-X-D-X- (59)

où D désigne un radical

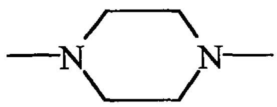

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :

(i)parmi les cationiques :

- l'homopolymère de chlorure de diméthyldiallylammonium vendu sous la dénomination MERQUAT 100DRY par la société MERCK;
- les copolymères de chlorure de diméthyldiallylammonium et d'acrylamide vendus sous la dénomination MERQUAT 2200 par la société CALGON;
- les polymères de type poly(ammonium quaternaire) préparés et décrits dans le brevet français 2 270 846, constitués de motifs récurrents de formules (W) et (U) suivantes :

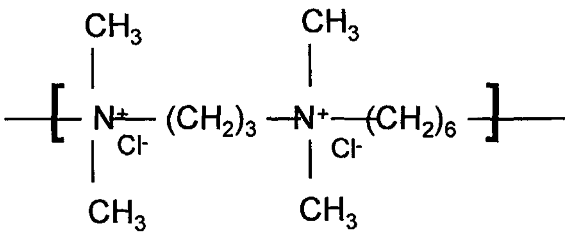 **(W)**

et notamment ceux dont le poids moléculaire, déterminé par chromatographie par perméation de gel, est compris entre 9500 et 9900;

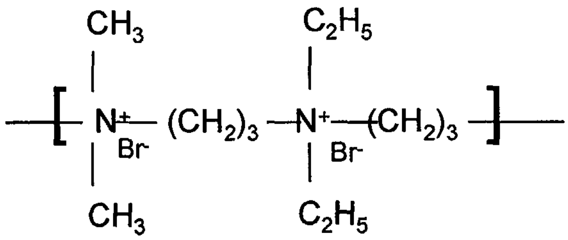 **(U)**

et notamment ceux dont le poids moléculaire, déterminé par chromatographie par perméation de gel, est d'environ 1200;

- les polymères de type poly(ammonium quaternaire) de la famille (11) et de formule (50) dans laquelle $X^-$ désigne le Chlore, et notamment ceux dont la masse moléculaire moyenne en poids est inférieure à 100 000, de préférence inférieure ou égale à 50 000;

(ii)parmi les polymères amphotères:

- le copolymère chlorure de diméthyldiallylammonium/acide acrylique (80/20) commercialisé sous la dénomination MERQUAT 280 DRY par la société CALGON (dénomination CTFA : POLYQUATERNIUM 22);
- le copolymère chlorure de diméthyldiallylammonium/acide acrylique (95/5) commercialisé sous la dénomination MERQUAT 295 DRY par la société CALGON (dénomination CTFA : POLYQUATERNIUM 22);
- le copolymère de chlorure de méthacrylamidopropyltrimonium, d'acide acrylique et d'acrylate d'éthyle, commercialisé sous la dénomination MERQUAT 2001 par la société CALGON (dénomination CTFA : POLYQUATERNIUM 47); et
- le terpolymère acrylamide/chlorure de diméthyldiallylammonium/acide acrylique, commercialisé sous la dénomination MERQUAT PLUS 3330 DRY par la société CALGON (dénomination CTFA : POLYQUATERNIUM 39).

Lorsqu'ils ils sont présents dans les pâtes anhydres de décoloration et de coloration simultanée de la présente invention, les polymères cationiques et/ou amphotères sont présents dans une proportion pondérale inférieure ou égale à 20% par rapport au poids total de ladite pâte et de préférence inférieure ou égale à 8%.

Par ailleurs, pour localiser le produit de décoloration et de coloration à l'application sur les cheveux afin qu'il ne coule pas sur le visage ou en dehors des zones que l'on se propose de traiter, il est d'usage d'épaissir ou de gélifier les compositions de décoloration avec des épaississants traditionnels tels que des polymères épaississants hydrosolubles comme les dérivés de cellulose, les dérivés d'amidon, l'acide polyacrylique réticulé, les alginates ou encore avec des silices épaississantes.

Les compositions anhydres pâteuse pour la décoloration et la coloration simultanée selon l'invention peuvent comprendre en outre au moins un agent gélifiant choisi dans le groupe formé par les silices pyrogénées à caractère hydrophile ou hydrophobe et les polymères blocs comprenant au moins un motif alkylène ou oxyde d'alkylène.
Parmi les silices pyrogénées à caractère hydrophile utilisables selon la présente invention, on peut citer notamment celles vendues par la société DEGUSSA HÜLS sous les dénominations commerciales AEROSIL® 90, 130, 150, 200, 300 et 380.

Parmi les silices pyrogénées à caractère hydrophobe utilisables selon la présente invention, on peut citer notamment celles vendues par la société DEGUSSA HÜLS sous les dénominations commerciales AEROSIL® R202, R805, R812, R972 et R974.
Parmi les polymères blocs comprenant au moins un motif alkylène ou oxyde d'alkylène, on peut citer notamment les copolymères di-, tri-, multi- ou radial blocs composés de segments de type styrène monomère et de segments de type monomère ou comonomère thermoplastique décrits dans le brevet US-5 221 534.

Parmi ces copolymères blocs, on préfère utiliser notamment ceux pour lesquels le monomère ou comonomère thermoplastique désigne éthylène/alkylène en $C_3$-$C_4$, et plus particulièrement encore un copolymère hydrogéné à blocs styrène et à blocs éthylène/alkylène en $C_3$-$C_4$.

On utilise plus avantageusement encore selon l'invention, un mélange de copolymère hydrogéné à blocs butylène/éthylène et à blocs styrène et de copolymère hydrogéné à blocs éthylène/propylène et à blocs styrène, dans de l'huile minérale, et notamment un mélange de 1 à 20% en poids de copolymère hydrogéné à blocs butylène/éthylène et à blocs styrène et de copolymère hydrogéné à blocs éthylène/propylène et à blocs styrène dans 80 à 99% en poids d'huile minérale.

De tels mélanges sont par exemple vendus par la société PENRECO sous les dénominations commerciales VERSAGEL® M200 et GEAHLENE® 200 et VERSAGEL® M750 et GEAHLENE® 750, ou par la société AIGLON sous les dénominations commerciales TRANSGEL® ou SYNGEL® ( 90% d'huile de paraffine, 5% de copolymère hydrogéné butylène/éthylène/styrène, 5% de copolymère hydrogéné éthylène/propylène/styrène).

On peut également utiliser les polymères tri-blocs styrène/éthylene-butylène/styrène (nom I.NC.I. "Hydrogenated styrene/butadiene copolymer") vendus par la société SHELL CHIMIE sous les dénominations commerciales KRATON® G-1650, G-1652 et G-1657.

Dans les compositions pâteuses selon l'invention, le ou les agents gélifiants sont de préférence présents à une concentration allant d'environ 0,01 à 10%, de préférence d'environ 0,01 à 5% et plus particulièrement d'environ 0,1 à 2,5% en poids par rapport au poids total de la composition.

Les compositions anhydres pâteuse pour la décoloration et la coloration simultanée selon l'invention peuvent comprendre en outre au moins un polymère épaississant hydrosoluble.

Polymères épaississants hydrosolubles

Selon l'invention, ils englobent tous les polymères hydrosolubles synthétiques ou d'origine naturelle utilisés classiquement dans le domaine cosmétique et différents des polymères amphiphiles non ioniques et/ou anioniques comportant au moins une chaîne grasse de la présente invention décrits ci-dessus.

Comme exemples de polymères synthétiques, on peut citer, la polyvinylpyrrolidone, l'acide polyacrylique, le polyacrylamide, l'acide poly-2-acrylamidopropanesulfonique non réticulé comme par exemple le produit vendu sous la dénomination SIMUGEL EG par la société SEPPIC, l'acide poly-2-acrylamido-2-méthylpropane sulfonique réticulé, l'acide poly-2-acrylamido-2-méthylpropane sulfonique réticulé et partiellement neutralisé par l'ammoniaque vendu sous la marque HOSTACERIN AMPS par la société CLARIANT, des mélanges à effet épaississant synergique de l'acide poly-2-acrylamido-2-méthylpropane sulfonique non réticulé avec des éthers d'hydroxyalkylcellulose ou avec des poly (oxyde d'éthylène) tel qu'ils sont décrits dans le brevet US-4540510, ou des mélanges à effet épaississant synergique d'un acide poly(méth)acrylamido-alkyl($C_1$-$C_4$)-sulfonique de préférence réticulé avec un copolymère réticulé de l'anhydride maléique et d'un alkyl($C_1$-$C_5$)vinyléther tels que le mélange HOSTACERIN AMPS / STABILEZE QM (de la société ISF) et tels qu'ils sont décrits dans la demande de brevet français de la demanderesse N°-0014416.

Les polymères épaississants d'origine naturelle utilisables selon la présente invention, sont de préférence des polymères comportant au moins un motif sucre, à savoir : les gommes de guar non-ioniques; les gommes de biopolysaccharides d'origine microbienne telles que les gommes de Scléroglucane ou de Xanthane; les gommes issues d'exudats végétaux telles que les gommes Arabique, Ghatti, Karaya, Tragacanthe, Carrageenane, Agar et Caroube; les pectines; les alginates; les amidons; les hydroxyalkyl($C_1$-$C_6$)celluloses et carboxyalkyl($C_1$-$C_6$)celluloses.

Par "motif sucre", on désigne au sens de la présente invention, une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

Les gommes de guar non ioniques peuvent être modifiées ou non modifiées.
Les gommes de guar non modifiées sont par exemple les produits vendus sous la dénomination GUARGEL D/15 par la société GOODRICH, VIDOGUM GH 175 par la société UNIPECTINE, et sous les dénominations MEYPRO-GUAR 50 et JAGUAR C par la société MEYHALL.
Les gommes de guar non-ioniques modifiées sont notamment modifiées par des groupements hydroxyalkyle en $C_1$-$C_6$. Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Les gommes de biopolysaccharides d'origine microbienne telles que les gommes de scléroglucane ou de xanthane, les gommes issues d'exudats végétaux telles que les gommes Aabique, gomme Ghatti, gomme Karaya, Tragacanthe, Carrageenane, Agar et Caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).

Parmi ces gommes, les scléroglucanes sont représentés par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.
Les xanthanes sont représentés par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.
Parmi les dérivés d'amidon, on peut citer par exemple le produit vendu sous la dénomination PRIMOGEL par la société AVEBE.

Les hydroxyalkyl($C_1$-$C_6$)celluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE

HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST.
Les hydroxyalkyl($C_1$-$C_6$)celluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON.
Parmi les carboxyalkyl($C_1$-$C_6$)celluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF , BLANOSE 7H, BLANOSE 7M31, BLANOSE 7H3SXF, par la société AQUALON, ou encore AQUASORB A500 et AMBERGUM 1221 par le société HERCULES, ou encore CELLOGEN HP810A et CELLOGEN HP6HS9, par la société MONTELLO, ou encore PRIMELLOSE par la société AVEBE.

Lorsqu'ils ils sont présents dans les compositions anhydres pâteuses de la présente invention, les polymères épaississants hydrosolubles sont présents dans une proportion pondérale allant d'environ 0,01 à 30% et de préférence d'environ 0,01 à 15% par rapport au poids total de la pâte.

<u>Autres adjuvants</u>

La composition anhydre pâteuse pour la décoloration et la coloration simultanée selon l'invention peut en outre contenir des cires hydrocarbonées, fluorées ou siliconées ou leurs mélanges. Les cires siliconées peuvent être des cires comportant une structure siliconée et des motifs à une ou plusieurs chaînes alkyles ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester. Comme autres cires utilisables dans l'invention, on peut citer les cires d'origine animale comme la lanoline, la cire d'abeille ; les cires d'origine végétale telles que la cire de carnauba ou de candellila, ; les cires d'origine minérale, par exemple de paraffine, de lignite ou les cires microcristallines, la cérésine ou l'ozokérite ; les cires synthétiques comme les cires de polyéthylène ; leurs mélanges.

De préférence, les compositions selon l'invention peuvent contenir de la cire d'abeille.

La composition anhydre pâteuse selon l'invention peut contenir en outre des charges telles que des argiles ou de la silice amorphe, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs anioniques, non ioniques, cationiques ou amphotères.

Les compositions de l'invention comprennent de préférence un ou plusieurs tensioactifs.
Le ou les tensioactifs peuvent être indifféremment choisis, seuls ou en mélanges, au sein des tensioactifs anioniques, amphotères, non ioniques, zwittérioniques et cationiques.
Les tensioactifs convenant à la mise en oeuvre de la présente invention sont notamment les suivants :

(i) <u>Tensioactif(s) anionique(s)</u> :
A titre d'exemple de tensio-actifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrates et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D-galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensioactif(s) amphotère(s) ou zwittérionique(s) :

Les agents tensioactifs amphotères ou zwitterioniques peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-CONHCH}_2\text{CH}_2\text{-N}(R_3)(R_4)(\text{CH}_2\text{COO}^-)$$

dans laquelle :

$R_2$ désigne un radical alkyle linéaire ou ramifié en $C_5$-$C_{20}$ provenant d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-CONHCH}_2\text{CH}_2\text{-N(B)(C)}$$

dans laquelle :

B représente $-CH_2CH_2OX'$, C représente $-(CH_2)_z-Y'$, avec z = 1 ou 2,

X' désigne le groupement $-CH_2CH_2-COOH$ ou un atome d'hydrogène

Y' désigne -COOH ou le radical $-CH_2-CHOH-SO_3H$

$R_2'$ désigne un radical alkyle linéaire ou ramifié, saturé ou non en $C_5$-$C_{20}$ provenant d'un acide $R'_2$-COOH présent par exemple dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et

de préférence de 0,5 à 30% du poids total de la composition.
Le pH de la composition de décoloration et coloration prête à l'emploi est généralement compris entre 4 et 12, de préférence entre 7 et 11,5, et plus particulièrement entre 8 et 11.
L'invention a pour autre objet une composition anhydre pâteuse prête à l'emploi destinée à la décoloration et la coloration simultanée des fibres kératiniques humaines, et en particulier des cheveux.
Par "composition prête à l'emploi", on entend, au sens de l'invention, la composition destinée à être appliquée telle quelle sur des fibres kératiniques, c'est à dire qu'elle résulte du mélange extemporané de la pâte et de la composition aqueuse de peroxyde d'hydrogène.
La composition anhydre pâteuse prête à l'emploi selon l'invention est obtenue par mélange au moment de l'emploi, de ladite composition pâteuse avec une composition aqueuse de peroxyde d'hydrogène ou par mélange au moment de l'emploi, de ladite composition pâteuse elle-même obtenue par mélange d'une composition A anhydre de décoloration sous forme de pâte contenant au moins un sel peroxygéné et au moins un agent alcalin et d'une composition B contenant au moins un colorant direct cationique, avec une composition aqueuse de peroxyde d'hydrogène.
L'invention concerne également un procédé de décoloration et de coloration simultanée des fibres kératiniques humaines, en particulier les cheveux.
Dans un premier temps, on mélange, avant l'emploi, une composition anhydre de décoloration et de coloration sous forme de pâte décrite ci-dessus avec une composition aqueuse de peroxyde d'hydrogène.
On applique le mélange obtenu sur la zone des fibres à traiter.
Puis on laisse poser pendant un temps généralement compris entre 3 et 60 minutes, de préférence entre 5 et 40 minutes.
Enfin, on élimine le mélange par rinçage à l'eau suivi d'un lavage avec un shampooing, puis éventuellement d'un séchage.
Un autre objet de l'invention porte sur un dispositif à plusieurs compartiments, ou "kit", pour la décoloration et la coloration des fibres kératiniques humaines et plus particulièrement des cheveux, comportant au moins deux compartiments, et de préférence deux dont l'un contient une composition anhydre pâteuse colorante selon l'invention, et l'autre contient une composition aqueuse de peroxyde d'hydrogène.
Un dispositif peut comporter au moins trois compartiments, et de préférence trois, dont l'un contient une composition A anhydre de décoloration pâteuse contenant au moins un sel peroxygéné, au moins un agent alcalin et au moins un liquide inerte, un second compartiment contient une composition B contenant au moins un colorant direct cationique, et le troisième compartiment renferme une composition aqueuse de peroxyde d'hydrogène.
Les exemples suivants de compositions de décoloration et de coloration simultanée sont destinés à illustrer l'invention sans présenter un caractère limitatif.

Exemple 1

Le tableau suivant comporte les compositions de 5 pâtes anhydres conformes à l'invention.

| | Quantités (en g% de matières première) | | | | | |
|---|---|---|---|---|---|---|
| | A | B | C | D | E | F |
| Persulfate de Potassium | 30 | 40 | 40 | 40 | 40 | 40 |
| Persulfate de Sodium | 12 | / | / | / | / | / |
| Disilicate de Sodium | 7,5 | 7,5 | 7,5 | 7,5 | 7,5 | 7,5 |
| Métasilicate de Sodium | 6,9 | 6,9 | 6,9 | 6,9 | 6,9 | 6,9 |
| Carbonate de Magnésium | 1,6 | 3,6 | 5,4 | 3,6 | 3,6 | 3,6 |
| Chlorure d'ammonium | 4,2 | 4,2 | 4,2 | 4,2 | 4,2 | 4,2 |
| EDTA | 1 | 1 | 1 | 1 | 1 | 1 |
| Copolymère hexaméthyldiisocyanate/polyéthylèneglycol à terminaison et stéarylpolyoxyéthylène, vendu sous la dénomination SER-AD FX 1100 par la société SERVO DELDEN | 2 | 2 | 2 | 2 | 2 | 2 |
| Carboxyméthyl amidon de pomme de terre / sel de sodium faiblement réticulé | 2 | 2 | 2 | 2 | 2 | 2 |
| Lauryl sulfate de Sodium | 4 | 4 | 4 | 4 | 4 | 4 |

(suite)

| | Quantités (en g% de matières première) | | | | | |
|---|---|---|---|---|---|---|
| | A | B | C | D | E | F |
| Stéarate de Magnésium | 2 | 2 | 2 | 2 | 2 | 2 |
| Silice amorphe | 1 | 1 | / | / | / | / |
| Silice hydrogénée à caractère hydrophile | / | / | 0,7 | / | 0,5 | 0,7 |
| Polydécène hydrogéné vendu sous la dénomination SILKFLO 366 NF POLYDECENE par la société AMOCO CHEMICAL | / | / | / | / | 23,8 | 23,8 |
| Silice pyrogénée à caractère hydrophobe | / | / | / | 0,5 | 0,5 | / |
| Myristate d'isopropyle | 21,6 | 22,6 | 23,3 | 23,5 | / | / |
| Cire d'abeille | 1,2 | 1,2 | / | / | / | / |
| Basic Red 51 | / | 2 | / | 2 | / | 1,3 |
| Basic Orange 31 | 2 | / | / | / | / | 1 |
| Basic Blue 41 | / | / | 1 | / | / | / |
| Basic Red 22 | / | / | / | / | 2 | / |
| Basic Yellow 67 | / | / | / | 0,8 | / | / |

Exemple 2

Le tableau suivant comporte :
- la composition B conforme à l'invention,
- la composition Bbis, identique à la composition B, mais ne contenant pas de colorant direct,
- la composition G, une composition pulvérulente conforme à l'art antérieur,
- la composition Gbis, une composition pulvérulente conforme à l'art antérieur, mais ne contenant pas de colorant direct.

| | Quantités (en g% de matières première) | | | |
|---|---|---|---|---|
| | B (invention ) | B bis | G (composition pulvérulente) | G bis |
| Persulfate de Potassium | 40 | 40 | 25 | 25 |
| Persulfate de Sodium | / | / | 25 | 25 |
| Disilicate de Sodium | 7,5 | 7,5 | / | / |
| Métasilicate de Sodium | 6,9 | 6,9 | 12 | 12 |
| Carbonate de Magnésium | 3,6 | 3,6 | 17,4 | 17,4 |
| Chlorure d'ammonium | 4,2 | 4,2 | / | / |
| Sulfate d'ammonium | / | / | 4,5 | 4,5 |
| Phosphate diammonique | / | / | 4 | 4 |
| EDTA | 1 | 1 | 2 | 2 |
| Copolymère hexaméthyldiisocyanate / polyéthylèneglycol à terminaison et stéarylpolyoxyéthylène, vendu sous la dénomination SER-AD FX 1100 par la société SERVO DELDEN | 2 | 2 | / | / |
| Carboxyméthyl amidon de pomme de terre / sel de sodium faiblement réticulé | 2 | 2 | / | / |
| Gomme de Guar | / | / | 2 | 2 |

(suite)

| | Quantités (en g% de matières première) | | | |
|---|---|---|---|---|
| | B (invention ) | B bis | G (composition pulvérulente) | G bis |
| Lauryl sulfate de Sodium | 4 | 4 | 3 | 3 |
| Stéarate de Magnésium | 2 | 2 | / | / |
| Silice | 1 | 3 | 3,1 | 5,1 |
| Silice hydrogénée à caractère hydrophile | / | / | 0,7 | / |
| Myristate d'isopropyle | 22,6 | 22,6 | / | / |
| Cire d'abeille | 1,2 | 1,2 | / | / |
| Basic Red 51 | 2 | / | 2 | / |

Les compositions B, Bbis, G et Gbis sont mélangées dans un rapport 1 + 1,5 avec une composition aqueuse de peroxyde d'hydrogène à 12% puis les compositions prêtes à l'emploi ainsi formées sont appliquées sur 3 mèches de cheveux châtain avec un rapport de bain de 10, pendant un temps de pose de 30 minutes, à une température de 40°C ± 2°C. Les mèches après traitement ont été ensuite rincées à l'eau, shampooinées puis séchées.
La demanderesse a effectué des mesures colorimétriques sur les 3 mèches de cheveux.

Le pouvoir décolorant des compositions Bbis et Gbis a été mesuré. L'éclaircissement a été lu avec un colorimètre MINOLTA CM 2002 dans le système international CIE L*a*b*. Chaque test d'éclaircissement a été effectué 3 fois pour pouvoir évaluer l'écart moyen de couleur ΔE et l'écart type par rapport à la mèche témoin châtain non décolorée.
L'écart de couleur ΔE est calculé en appliquant l'équation suivante :

$$\Delta E = \sqrt{(L^* - L_0^*)^2 + (a^* - a_0^*)^2 + (b^* - b_0^*)^2}$$

Dans cette équation, ΔE représente la différence de couleur entre la mèche décolorée et la mèche témoin, L*, a* et b* représentent respectivement les mesures de la mèche décolorée, $L_0^*$, $a_0^*$ et $b_0^*$ représentant respectivement les mesures de la mèche témoin châtain.
Dans le tableau ci-dessous réunissant les résultats de mesure, plus la valeur de ΔE est grande, plus la différence de couleur entre les deux mèches est grande, et plus l'éclaircissement est important.

| | Composition Bbis | Composition Gbis |
|---|---|---|
| Eclaircissement ΔE | 43,0<br>écart type 1,7 | 41,6<br>écart type 2,6 |

Ces résultats montrent que les performances éclaircissantes ou décolorantes obtenues avec la pâte de l'invention sont équivalentes à celles de l'art antérieur.

Le pouvoir colorant des compositions B et G a été mesuré. La chromaticité a été lue avec un colorimètre MINOLTA CM 2002 dans le système international CIE L*a*b* et dans le système TSL (Teinte, Saturation, Luminosité) ou L*C*h.

La chromaticité est calculée selon la formule :

$$C^* = \sqrt{a^{*2} + b^{*2}}$$

Les résultats figurent dans le tableau ci-dessous :

| | Composition B | Composition G |
|---|---|---|
| Chromaticité C* | 33,7<br>écart type 1,2 | 28,2<br>écart type 1,6 |

La composition prête à l'emploi issue de la composition B, conforme à l'invention donne des mèches rouges, très chromatiques. En revanche, la composition prête à l'emploi issue de la composition G, conforme à l'art antérieur donne des mèches rouge acajou, moins chromatiques.

Ces résultats montrent que la composition B offre des performances colorantes bien supérieures à celles de l'art antérieur.

## Revendications

**1.** Composition anhydre et pâteuse, pouvant contenir jusqu'à 1% en poids d'eau pour la décoloration et la coloration simultanée des fibres kératiniques humaines, en particulier les cheveux, comprenant dans un milieu approprié pour la teinture

- au moins un sel peroxygéné,
- au moins un agent alcalin,
- 15 à 35% en poids d'au moins un liquide organique inerte par rapport au poids total de la composition choisi dans le groupe formé par les polydécènes de formule $C_{10n}$ $H_{[(20n)+2]}$ dans laquelle n varie de 3 à 9, les esters d'alcools gras ou d'acides gras, les esters ou di-esters de sucres d'acides gras en $C_{12}$-$C_{24}$, les éthers cycliques ou les esters cycliques, les huiles de silicone, les huiles minérales ou les huiles végétales.
- au moins un colorant direct cationique.

**2.** Composition selon la revendication 1, **caractérisée par le fait que** le colorant direct cationique est choisi parmi les colorants xanthèniques, azoïques, azométhiniques et méthiniques.

**3.** Composition selon la revendication 1 ou 2, **caractérisée par le fait que** le colorant direct cationique est un colorant direct hétérocyclique.

**4.** Composition selon la revendication 3, **caractérisé par le fait que le** colorant direct possède au moins une charge cationique sur un hétérocycle.

**5.** Composition selon la revendication 4, **caractérisée par le fait que** le colorant direct est un colorant direct azoïque, azométhinique ou méthinique possédant au moins une charge cationique sur un hétérocycle.

**6.** Composition selon l'une quelconque des revendications 2 à 5, **caractérisée par le fait que** le colorant direct cationique est choisi parmi le groupe de colorants formé par: le Acid Red 52, le Basic Blue 41, le Basic Blue 67, le Basic Brown 1, le Basic Brown 4, le Basic Red 18, le Basic Red 22, le Basic Red 46, le Basic Red 104, le Basic Violet 35, le Basic Yellow 45, le Basic Yellow 57, le Basic Yellow 67, le Basic Red 14. le Basic Yellow 13 et le Basic Yellow 29 ainsi que les colorants suivants :

- les colorants de formule (1):

$$G\text{-}N{=}{=}N\text{ -}J \qquad (I)$$

dans laquelle

le symbole G représente un groupement choisi parmi les structures $G_1$ à $G_3$ suivantes :

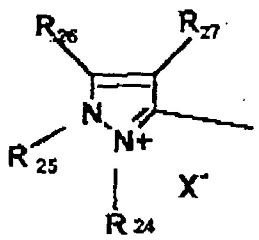

$G_1$

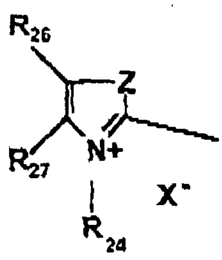

$G_2$

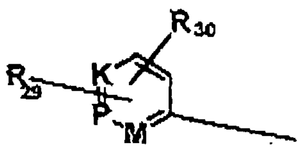

$G_3$

dans lesquelles,

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;

$R_{25}$ désigne un radical alkyle en $C_1$-$C_4$ ou un radical phényle;

$R_{26}$ et $R_{27}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$, alcoxy en $C_1$-$C_4$, ou $NO_2$, au forment ensemble dans $G_2$ un cycle benzénique éventuellement substitué par un ou plusieurs radicaux alkyle en $C_1$-$C_4$ alcoxy en $C_1$-$C_4$, ou $NO_2$;

$R_{26}$ peut désigner en outre un atome d'hydrogène,

Z désigne un atome d'oxygène, de soufre ou un groupement $-NR_{25}$;

M représente un groupement -CH, -CR (R désignant alkyle en $C_1$-$C_4$), ou $-NR_{28}(X^-)_r$;

K représente un groupement -CH, -CR (R désignant alkyle en $C_1$-$C_4$), ou - $NR_{28}(X^-)_r$:

P représente un groupement -CH, -CR (R désignant alkyle en $C_1$-$C_4$),ou - $NR_{28}(X^-)_r$;

r désigne zéro ou 1;

$R_{28}$ représente un atome $O^-$, un radical alcoxy en $C_1$-$C_4$, ou un radical alkyle en $C_1$-$C_4$;

$R_{29}$ et $R_{30}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical $-NO_2$:

$X^-$ représente un anion de préférence choisi parmi le chlorure, l'iodure, le méthyl sulfate, l'ethyl sulfate, l'acétate et le perchlorate;

le symbole J représente :

- (a) un groupement de structure $J_1$ suivante :

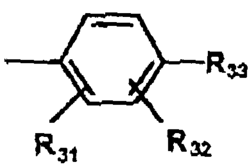

$J_1$

dans laquelle.

$R_{31}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{34}$, $-NR_{35}R_{36}$, -NHCOalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre;

$R_{32}$ représente un atome d'hydrogène, un atome d'halogène choisi parmi le chlore, le brome, l'iode et le fluor, un radical alkyle en $C_1$-$C_4$, alcoxy en $C_1$-$C_4$, ou forme avec $R_{33}$ ou $R_{34}$ un cycle à 5 ou 6 chaînons contenant ou non un ou plusieurs hétéroatomes choisis parmi l'azote, l'oxygène, ou le soufre;

$R_{33}$ représente un atome d'hydrogène, un radical -OH, un radical - $NHR_{34}$, un radical $-NR_{35}R_{36}$;

$R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;

$R_{35}$ et $R_{36}$, identiques ou différents, représentent un radical alkyle en $C_1$-$C_4$, un radical monohydroxyalkyle en $C_2$-$C_4$, polyhydroxyalkyle en $C_2$-$C_4$;

- (b) un groupement hétérocyclique azoté à 5 ou 6 chaînons susceptible de renfermer d'autres hétéroatomes et/ou des groupements carbonylés et pouvant être substitué par un on plusieurs radicaux alkyle en $C_1$-$C_4$, amino ou phényle,

et notamment un groupement de structure $J_2$ suivante :

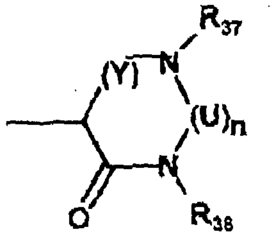

$J_2$

dans laquelle,

$R_{37}$ et $R_{38}$, identiques ou différents, représentent un atome d'hydrogène un radical alkyle en $C_3$-$C_{10}$, un radical phényle;

Y désigne le radical -CO- ou le radical $-C(CH_3)=$ ;

n représente 0 ou 1, avec, lorsque n désigne 1, U désigne le radical -CO-

- les composés de formule (II) suivante :

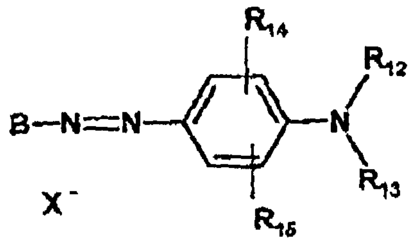

(II)

dans laquelle ;

$R_{12}$ représente un atome d'hydrogène ou un radical alkyle en $C_1$-$C_4$,

$R_{13}$, représente un atome d'hydrogène, un radical alkyle pouvant être substitué par un radical -CN ou par un groupement amino, un radical 4'-aminophényle ou forme avec $R_{12}$ un hétérocycle éventuellement oxygéné et/ou azoté pouvant être substitué par un radical alkyle en $C_1$-$C_4$.

$R_{14}$ et $R_{15}$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,

X représente un anion de préférence choisi parmi le chlorure, le méthyl sulfate et l'acétate,

B représente un groupement choisi par les structures B1 à B6 suivantes :

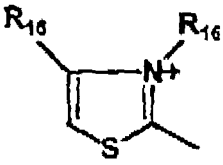 ; 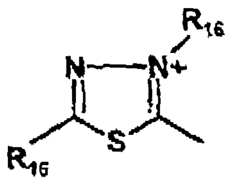 ; 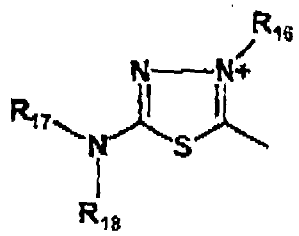 ;

B1 B2 B3

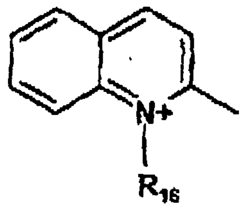 ; 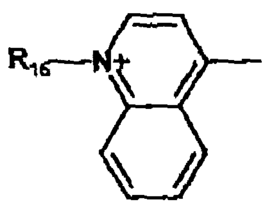 et 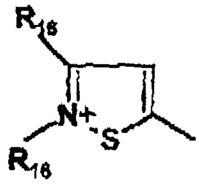 ;

B4 B5 B6

dans lesquelles $R_{16}$ représente un radical alkyle en $C_1$-$C_4$,
$R_{17}$ et $R_{18}$, identiques ou différents, représentent un atome d'hydrogène ou un radical alkyle en $C_1$-$C_4$
- les composés de formules (III) et (IV) suivantes :

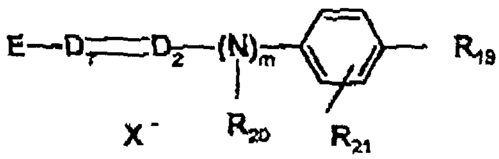

(III)

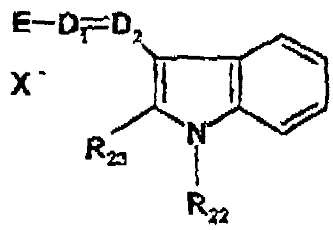

(IV)

dans lesquelles :

$R_{19}$ représente un atome d'hydrogène, un radical alcoxy en $C_1$-$C_4$, un atome d'halogène tel que le brome, le chlore, l'iode ou le fluor ou un radical amino,
$R_{20}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{21}$ représente un atome d'hydrogène ou d'halogène tel que le brome, le chlore, l'iode ou le fluor,
$R_{22}$ et $R_{23}$, identiques ou différents, représentent un atome d'hydrogène ou un radical alkyle en $C_1$-$C_4$,
$D_1$ et $D_2$, identiques ou différents, représentent un atome d'azote ou le groupement -CH,
m représente 0 ou 1,
étant entendu que lorsque $R_{19}$ représente un groupement amino non substitué, alors $D_1$ et $D_2$ représentent simultanément un groupement -CH et m = 0,
$X^-$ représente un anion de préférence choisi parmi le chlorure, le méthyl sulfate et l'acétate,

E représente un groupement choisi par les structures E1 à E8 suivantes :

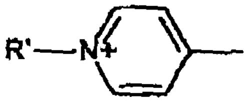

E1

;

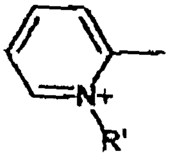

E2

;

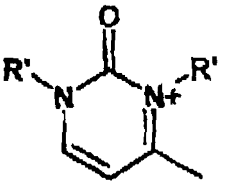

E3

;

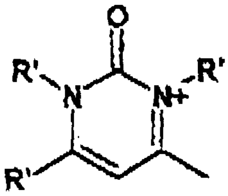

E4

;

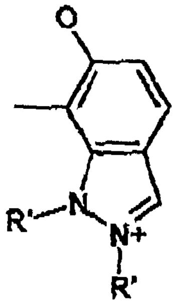

E5

;

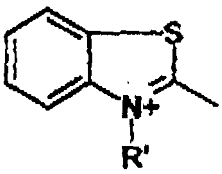

E6

;

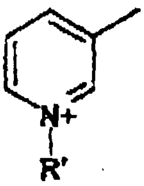

E7

et

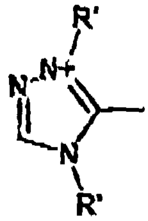

E8

dans lesquelles R' représente un radical alkyle en $C_1$-$C_4$ ;
lorsque m représente 0 et que $D_1$ représente un atome d'azote, alors E peut également désigner un groupement de structure E9 suivante :

E9

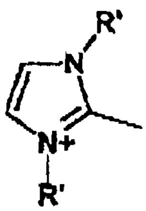

dans laquelle R' représente un radical alkyle en $C_1$-$C_4$.
- les colorants de formule (V)

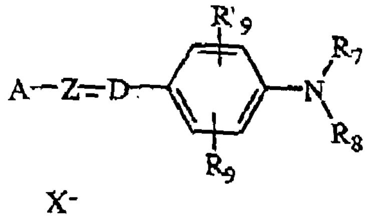

(V)

dans laquelle :

Z et D représentent, identiques ou différents, un atome d'azote ou le radical -CH-,
$R_7$ et $R_8$, identiques ou différents, représentent un atome d'hydrogène ; un radical alkyle en $C_1$-$C_4$ pouvant être substitué par un radical -CN,-
OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,
$R_9$ et $R'_9$, identiques ou différents, représentent un atome d'hydrogène ou d'halogène choisi parmi le chlore, le brome, l'iode et le fluor, un radical cyano, alkyl en $C_1$-$C_4$ alcoxy en $C_1$-$C_4$ ou acétyloxy,
X représente un anion de préférence choisi parmi le chlorure, le méthyl sulfate et l'acétate,

A représente un groupement choisi par les structures A1 à A19 suivantes

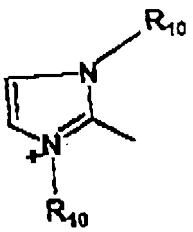

$A_1$ ;

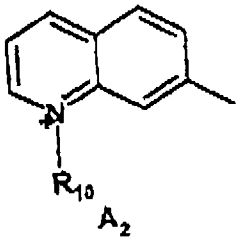

$A_2$ ;

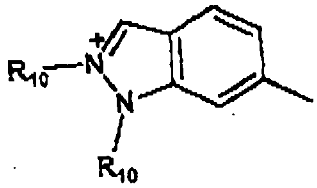

$A_3$ ;

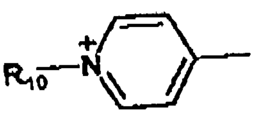

$A_4$ ;

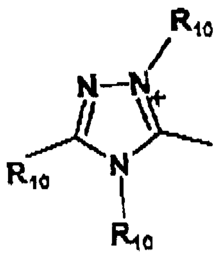

$A_5$ ;

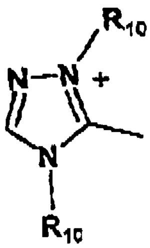

$A_6$ ;

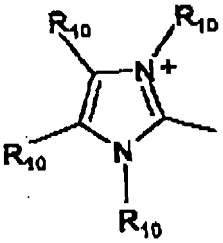

$A_7$

;

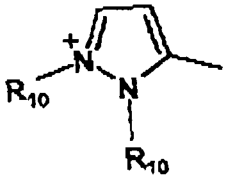

$A_8$

;

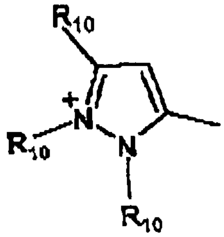

$A_9$

;

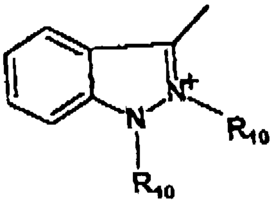

$A_{10}$

;

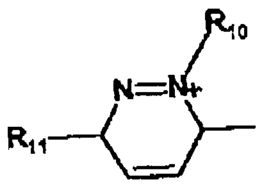

$A_{11}$

;

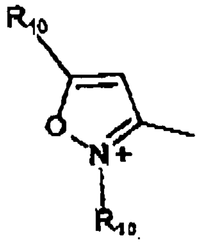

$A_{12}$

;

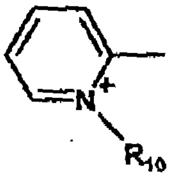

$A_{13}$

;

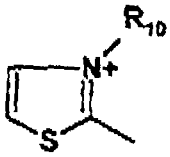

$A_{14}$

;

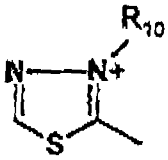

$A_{15}$

;

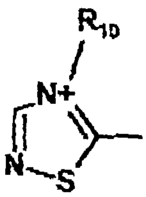

$A_{16}$

;

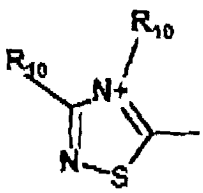

$A_{17}$

;

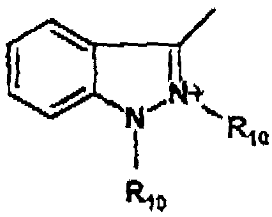

$A_{18}$

et

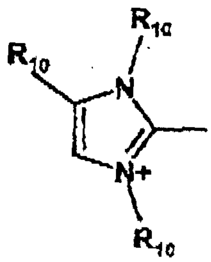

A19

dans lesquelles $R_{10}$ représente un radical alkyle en $C_1$-$C_4$ pouvant être substitué par un radical hydroxyle et $R_{11}$ représente un radical alcoxy en $C_1$-$C_4$,
les composés de formules (VI), (VII) et (VIII) :

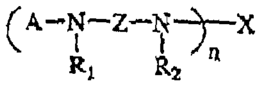

(VI)

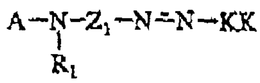

(VII)

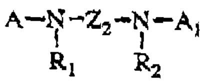

(VIII)

dans lesquelles
A et $A_1$, indépendamment l'un de l'autre, sont les restes de formule ;

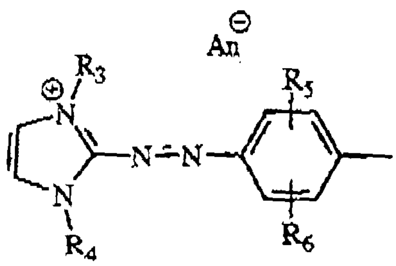

Z représente un reste de diamine aliphatique ou aromatique,
$R_1$ et $R_2$, indépendamment l'un de l'autre:, représentent un atome d'hydrogène, un groupe alkyle en $C_1$-$C_4$ ou peuvent former ensemble avec 2 atomes d'azote auxquels ils sont rattachés ou avec Z et $Z_2$ un cycle à 5, 6 ou 7 chaînons,
X représente le reste d'un chaînon formant un pont,
n représente un nombre entier 2, 3 ou 4,
$Z_1$ représente un reste de diamines aromatique,
$Z_2$ représente un reste de diamines aliphatique,
KK représente un reste de composé coupleur,
$R_3$ et $R_4$, indépendamment l'un de l'autre, représentent un atome d'hydrogène ou un groupe alkyle en $C_1$-$C_4$ ,
$R_5$ et $R_6$, indépendamment l'un de l'autre, représentent un atome d'hydrogène, un groupe alkyle en $C_1$-$C_4$ ou un groupe alkoxy en $C_1$-$C_4$,
An représente un anion incolore.

7. Composition selon l'une quelconque des revendications 1 à 6, **caractérisée par le fait que** le colorant direct cationique est choisi parmi le Basic Red 51, le Basic yellow 87 et le Basic Orange 31.

**8.** Composition selon l'une quelconque des revendications précédentes, **caractérisée en ce que** la concentration en colorant direct cationique est comprise entre 0,001% et 20%, et de préférence entre 0,01% à 10%, et plus préférentiellement de 0,1% à 5% en poids total de la composition.

**9.** Composition selon l'une quelconque des revendications précédentes, **caractérisée par le fait que** le sel peroxygéné est choisi parmi les persulfates, les perborates, les percarbonates et le peroxyde de métaux alcalins ou alcalino-terreux.

**10.** Composition selon la revendication 9, **caractérisée par le fait que** le sel peroxygéné est le persulfate de sodium ou le persulfate de potassium.

**11.** Composition selon l'une quelconque des revendications précédentes, **caractérisée en ce que** la concentration en sels peroxygénés est comprise entre 10% à 70%, et de préférence entre 20% à 60%, en poids total de la composition.

**12.** Composition selon l'une quelconque des revendications précédentes, **caractérisée en ce que** l'agent alcalin est choisi parmi l'urée, le chlorure d'ammonium, le sulfate d'ammonium, le phosphate d'ammonium ou le nitrate d'ammonium, les silicates, le phosphate ou le carbonate de métaux alcalins ou alcalino-terreux.

**13.** Composition selon l'une quelconque des revendications précédentes, **caractérisée en ce que** la concentration en agent alcalin est comprise entre 0,01% et 40%, et de préférence entre 0,1 à 30% en poids total de la composition.

**14.** Composition selon l'une quelconque des revendications précédentes, **caractérisée par le fait que** le ou les liquides organiques inertes sont choisis dans le groupe formé par les polydécènes de formule $C_{10n}$ $H_{[(20n)+2]}$ dans laquelle n varie de 3 à 9 et de préférence de 3 à 7, et les esters d'alcools gras ou d'acides gras.

**15.** Composition selon l'une quelconque des revendications précédentes, **caractérisée en ce qu**'elle comprend au moins un polymère amphiphile non ionique et/ou bionique comportant au moins une chaîne grasse.

**16.** Composition selon l'une quelconque des revendications précédentes, **caractérisée en ce que** la concentration en polymère amphiphile non ionique et/ou anionique comportant au moins une chaîne grasse est comprise entre 0,01% et 30%, et de préférence entre 0,01% à 15% en poids total de la composition.

**17.** Composition salon l'une quelconque des revendications précédentes, **caractérisée en ce qu**'elle comprend comme adjuvant de la cire d'abeille.

**18.** Composition prête à l'emploi de décoloration et de coloration simultanée des fibres kératiniques humaines, en particulier les cheveux, **caractérisée en ce qu**'elle résulte d'un mélange extemporanée d'une composition anhydre pâteuse telle que définie à l'une quelconque des revendications 1 à 17 et d'une composition comprenant du peroxyde d'hydrogène.

**19.** Composition prête à l'emploi selon la revendication 18,
**caractérisée par le fait que** ladite composition anhydre pâteuse comprend un mélange d'une composition A anhydre de décoloration sous forme de pâte contenant au moins un sel peroxygéné, au moins un agent alcalin et au moins un liquide organique inerte, et une composition B contenant au moins un colorant direct cationique.

**20.** Procédé de décoloration et de coloration simultanée des fibres kératiniques humaines, en particulier les cheveux, comprenant les étapes consistant :

- à mélanger, avant l'emploi, une composition anhydre de décoloration et de coloration sous forme de pâte définie selon l'une quelconque des revendications 1 à 17 avec une composition aqueuse de peroxyde d'hydrogène,
- à appliquer le mélange obtenu sur la zone des fibres à traiter,
- à laisser poser pendant un temps généralement compris entre 3 et 60 minutes, de préférence entre 5 et 40 minutes.
- à éliminer le mélange par rinçage à l'eau suivi d'un lavage avec un shampooing, puis éventuellement d'un séchage.

**21.** Dispositif à deux compartiments, ou kit, pour la décoloration et la coloration des fibres kératiniques humaines et

plus particulièrement des cheveux, **caractérisé par le fait que** le premier compartiment renferme une composition anhydre pâteuse telle que décrite dans l'une quelconque des revendications de 1 à 17, et le second compartiment renferme une composition aqueuse de peroxyde d'hydrogène.

**22.** Dispositif à plusieurs compartiments, ou kit, pour la décoloration et la coloration des fibres kératiniques humaines et plus particulièrement des cheveux, **caractérisé par le fait que** le premier compartiment renferme une composition A de décoloration anhydre et pâteuse, pouvant contenir jusqu'à 1% en poids d'eau, contenant au moins un sel peroxygéné, au moins un agent alcalin et au moins un liquide organique inerte, te second compartiment contient une composition B contenant au moins un colorant direct cationique, et le troisième compartiment renferme une composition aqueuse de peroxyde d'hydrogène.

## Claims

**1.** Pasty anhydrous composition possibly containing up to 1% by weight of water, for simultaneously bleaching and dyeing human keratin fibres, and in particular the hair, and comprising, in a medium that is suitable for dyeing:

- at least one peroxygenated salt,
- at least one alkaline agent,
- 15% to 35% by weight, relative to the total weight of the composition, of at least one inert organic liquid chosen from the group formed by polydecenes of formula $C_{10n}H_{[(20n)+2]}$ in which n ranges from 3 to 9, esters of fatty alcohols or of fatty acids, sugar esters or diesters of $C_{12}$-$C_{24}$ fatty acids, cyclic ethers or cyclic esters, silicone oils, mineral oils and plant oils, and
- at least one cationic direct dye.

**2.** Composition according to Claim 1, **characterized in that** the cationic direct dye is chosen from xanthene dyes, azo dyes, azomethine dyes and methine dyes.

**3.** Composition according to Claim 1 or 2, **characterized in that** the cationic direct dye is a heterocyclic direct dye.

**4.** Composition according to claim 3, **characterized in that** the direct dye has at least one cationic charge on a heterocycle.

**5.** Composition according to Claim 4, **characterized in that** the direct dye is an azo, azomethine or methine direct dye with at least one cationic charge on a heterocycle.

**6.** Composition according to any one of Claims 2 to 5, **characterized in that** the cationic direct dye is chosen from the group of dyes formed by: Acid Red 52, Basic Blue 41, Basic Blue 67, Basic Brown 1, Basic Brown 4, Basic Red 18, Basic Red 22, Basic Red 46, Basic Red 104, Basic Violet 35, Basic Yellow 45, Basic Yellow 57, Basic Yellow 67, Basic Red 14, Basic Yellow 13 and Basic Yellow 29, and also the following dyes:

- the dyes of formula (I):

$$G\text{-}N\text{=}N\text{-}J \qquad (I)$$

in which:

the symbol G represents a group chosen from the structures $G_1$ to $G_3$ below:

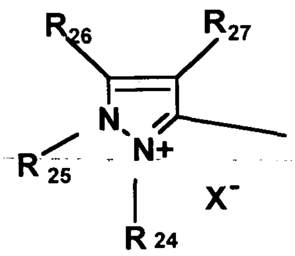

G$_1$

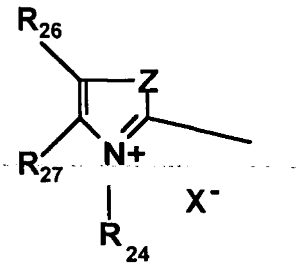

G$_2$

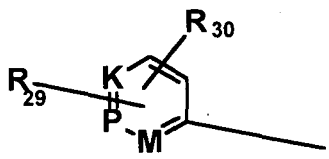

G$_3$

in which:

$R_{24}$ denotes a $C_1$-$C_4$ alkyl radical, a phenyl radical which may be substituted with a $C_1$-$C_4$ alkyl radical, or a halogen atom chosen from chlorine, bromine, iodine and fluorine;
$R_{25}$ denotes a $C_1$-$C_4$ alkyl radical or a phenyl radical;
$R_{26}$ and $R_{27}$, which may be identical or different, represent a $C_1$-$C_4$ alkyl radical, a phenyl radical, or form together in $G_1$ a benzene ring substituted with one or more $C_1$-$C_4$ alkyl, $C_1$-$C_4$ alkoxy or $NO_2$ radicals, or form together in $G_2$ a benzene ring optionally substituted with one or more $C_1$-$C_4$ alkyl, $C_1$-$C_4$ alkoxy or $NO_2$ radicals;
$R_{26}$ may also denote a hydrogen atom;
Z represents an oxygen or sulphur atom or a group $-NR_{25}$ ;
M represents a group -CH, -CR (R denoting $C_1$-$C_4$ alkyl) or $-NR_{28}$ $(X^-)_r$;
K represents a group -CH, -CR (R denoting $C_1$-$C_4$ alkyl) or $-NR_{28}$ $(X^-)_r$;
P represents a group -CH, -CR (R denoting $C_1$-$C_4$ alkyl) or $-NR_{28}$ $(X^-)_r$;
r denotes 0 or 1;
$R_{28}$ represents an $O^-$ atom, a $C_1$-$C_4$ alkoxy radical or a $C_1$-$C_4$ alkyl radical;
$R_{29}$ and $R_{30}$, which may be identical or different, represent a hydrogen atom or a halogen atom chosen from chlorine, bromine, iodine and fluorine, a $C_1$-$C_4$ alkyl or $C_1$-$C_4$ alkoxy radical, or an $-NO_2$ radical;
$X^-$ represents an anion preferably chosen from chloride, iodide, methyl sulphate, ethyl sulphate, acetate and perchlorate;

the symbol J represents:

- (a) a group of structure $J_1$ below:

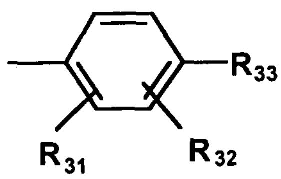

J$_1$

in which:

$R_{31}$ represents a hydrogen atom, a halogen atom chosen from chlorine, bromine, iodine and fluorine, a $C_1$-$C_4$ alkyl or $C_1$-$C_4$ alkoxy radical, an -OH, $-NO_2$, $-NHR_{34}$, $-NR_{35}R_{36}$ or $C_1$-$C_4$ -NHCOalkyl radical, or forms with $R_{32}$ a 5- or 6-membered ring optionally containing one or more hetero atoms chosen from nitrogen, oxygen and sulphur;

$R_{32}$ represents a hydrogen atom, a halogen atom chosen from chlorine, bromine, iodine and fluorine, a $C_1$-$C_4$ alkyl or $C_1$-$C_4$ alkoxy radical, or forms with $R_{33}$ or $R_{34}$ a 5- or 6-membered ring optionally containing one or more hetero atoms chosen from nitrogen, oxygen and sulphur;

$R_{33}$ represents a hydrogen atom, an -OH radical, a radical $-NHR_{34}$ or a radical $-NR_{35}R_{36}$;

$R_{34}$ represents a hydrogen atom, a $C_1$-$C_4$ alkyl radical, a $C_1$-$C_4$ monohydroxyalkyl or $C_2$-$C_4$ polyhydroxyalkyl radical or a phenyl radical;

$R_{35}$ and $R_{36}$, which may be identical or different, represent a $C_1$-$C_4$ alkyl radical or a $C_1$-$C_4$ monohydroxyalkyl or $C_2$-$C_4$ polyhydroxyalkyl radical;

- (b) a 5- or 6-membered nitrogenous heterocyclic group, which may contain other hetero atoms and/or carbonyl groups and may be substituted with one or more $C_1$-$C_4$ alkyl, amino or phenyl radicals, and especially a group of structure $J_2$ below:

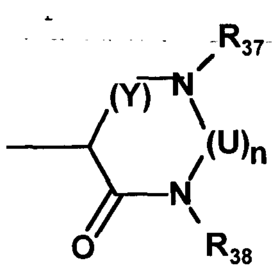

$J_2$

in which:

$R_{37}$ and $R_{38}$, which may be identical or different, represent a hydrogen atom, a $C_3$-$C_{10}$ alkyl radical or a phenyl radical;

Y denotes a -CO- radical or a $-C(CH_3)=$ radical;

n represents 0 or 1, with, when n denotes 1,U denotes a -CO- radical

- the compounds of formula (II) below:

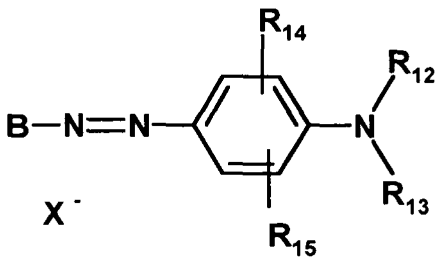

(II)

in which:

$R_{12}$ represents a hydrogen atom or a $C_1$-$C_4$ alkyl radical,

$R_{13}$ represents a hydrogen atom, an alkyl radical which may be substituted with a -CN radical or with an amino group, a 4'-aminophenyl radical, or forms with $R_{12}$ a heterocycle optionally containing oxygen and/or nitrogen, which may be substituted with a $C_1$-$C_4$ alkyl radical,

$R_{14}$ and $R_{15}$, which may be identical or different, represent a hydrogen atom, a halogen atom such as bromine, chlorine, iodine or fluorine, a $C_1$-$C_9$ alkyl or $C_1$-$C_4$ alkoxy radical, or a -CN radical,

$X^-$ represents an anion preferably chosen from chloride, methyl sulphate and acetate,

B represents a group chosen from structures B1 to B6 below:

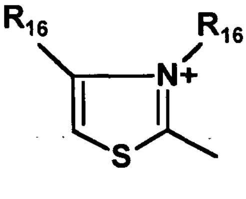 ; 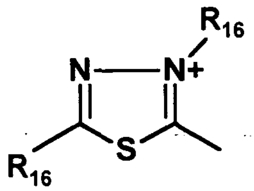 ; 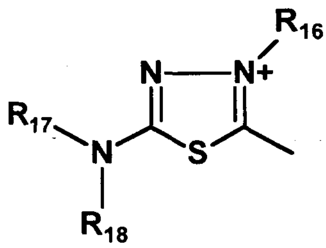 ;

B1 B2 B3

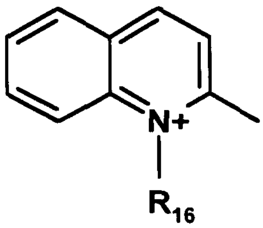 ; 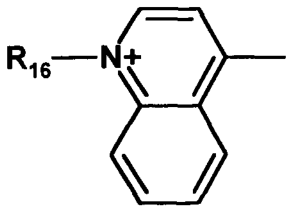 and 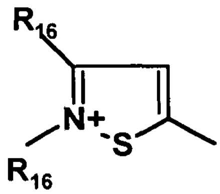 ;

B4 B5 B6

in which $R_{16}$ represents a $C_1$-$C_4$ alkyl radical,
$R_{17}$ and $R_{18}$, which may be identical or different, represent a hydrogen atom or a $C_1$-$C_4$ alkyl radical

- the compounds of formulae (III) and (IV) below:

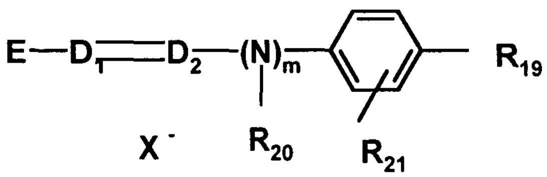

(III)

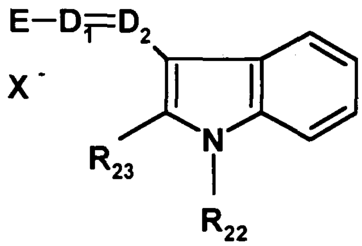

(IV)

in which:

$R_{19}$ represents a hydrogen atom, a $C_1$-$C_4$ alkoxy radical, a halogen atom such as bromine, chlorine, iodine or fluorine, or an amino radical,
$R_{20}$ represents a hydrogen atom, a $C_1$-$C_4$ alkyl radical or forms, with a carbon atom of the benzene ring, a heterocycle optionally containing oxygen and/or substituted with one or more $C_1$-$C_4$ alkyl groups,
$R_{21}$ represents a hydrogen atom or a halogen atom such as bromine, chlorine, iodine or fluorine,
$R_{22}$ and $R_{23}$, which may be identical or different, represent a hydrogen atom or a $C_1$-$C_4$ alkyl radical,
$D_1$ and $D_2$, which may be identical or different, represent a hydrogen atom or a -CH group,
m represents 0 or 1,
it being understood that when $R_{19}$ represents an unsubstituted amino group, then $D_1$ and $D_2$ simultaneously represent a -CH group and m = 0,
$X^-$ represents an anion preferably chosen from chloride, methyl sulphate and acetate,

E represents a group chosen from structures E1 to E8 below:

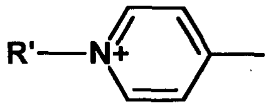

E1

;

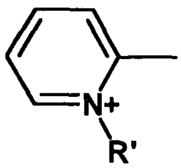

E2

;

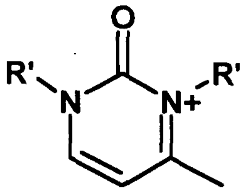

E3

;

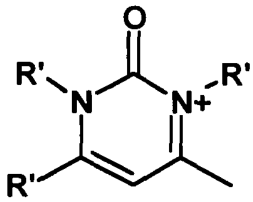

E4

;

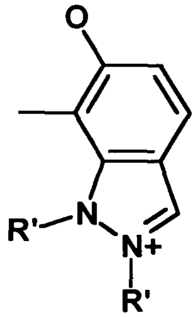

E5

;

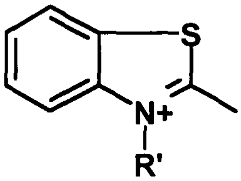

E6

;

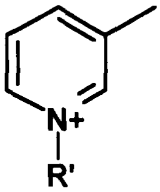

E7

and

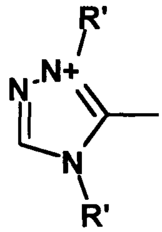

E8

in which R' represents a $C_1$-$C_9$ alkyl radical;
when m represents 0 and $D_1$ represents a nitrogen atom,
then E may also denote a group of structure E9 below:

E9

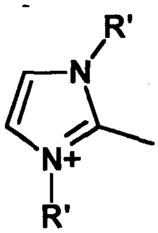

in which R' represents a $C_1$-$C_4$ alkyl radical

- the compounds of formula (V) below:

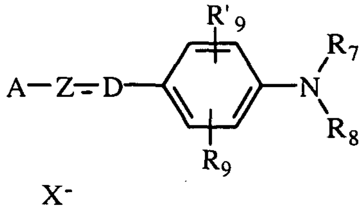

(V)

in which:

Z and D, which may be identical or different, represent a nitrogen atom or a -CH- radical,

$R_7$ and $R_8$, which may be identical or different, represent a hydrogen atom; a $C_1$-$C_4$ alkyl radical which may be substituted with a -CN, -OH or -$NH_2$ radical, or form, with a carbon atom of the benzene ring, a heterocycle optionally containing oxygen or nitrogen, which may be substituted with one or more $C_1$-$C_4$ alkyl radicals; a 4'-aminophenyl radical,

$R_9$ and $R'_9$, which may be identical or different, represent a hydrogen atom or a halogen atom chosen from chlorine, bromine, iodine and fluorine, or a cyano, $C_1$-$C_4$ alkyl, $C_1$-$C_4$ alkoxy or acetyloxy radical,

$X^-$ represents an anion preferably chosen from chloride, methyl sulphate and acetate,

A represents a group chosen from structures A1 to A19 below:

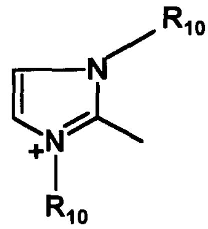

$A_1$ ;

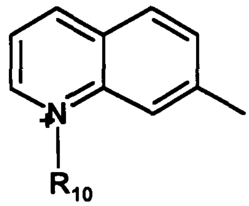

$A_2$ ;

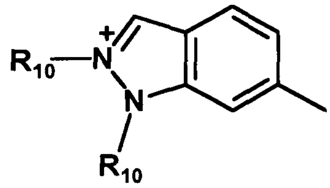

$A_3$

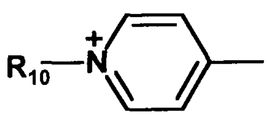

$A_4$ ;

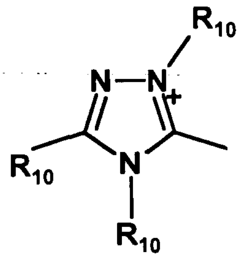

$A_5$ ;

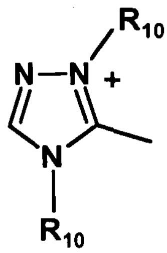

$A_6$ ;

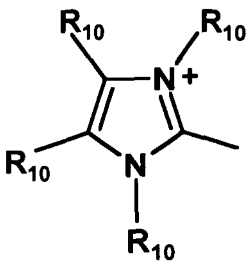
A7
;
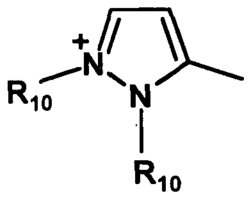
A8
;
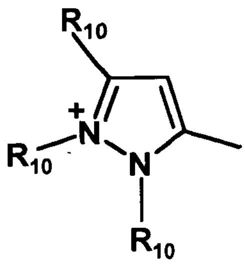
A9
;

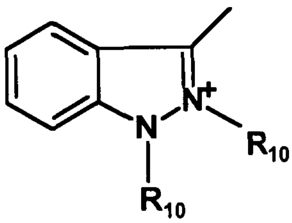
A10
;
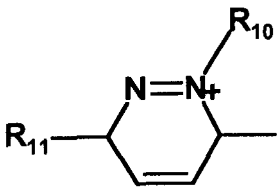
A11
;
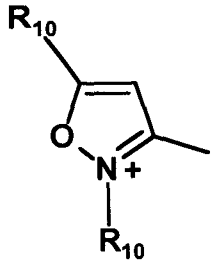
A12
;

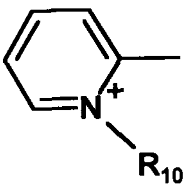
A13
;
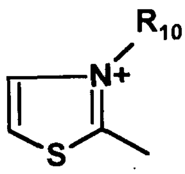
A14
;
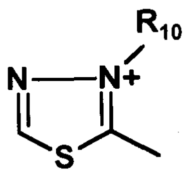
A15

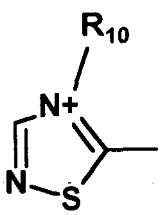
A16
;
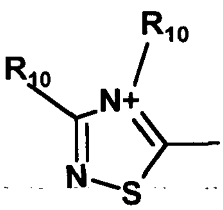
A17
;
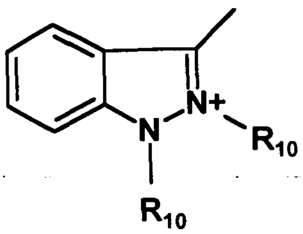
A18

and

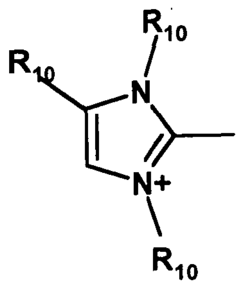

$A_{19}$

in which $R_{10}$ represents a $C_1$-$C_4$ alkyl radical which may be substituted with a hydroxyl radical and $R_{11}$ represents a $C_1$-$C_4$ alkoxy radical,

- the compounds of formulae (VI), (VII) and (VIII):

**(VI)** 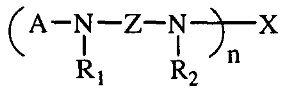

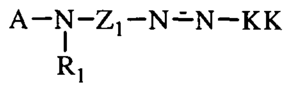 **(VII)**

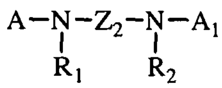 **(VIII)**

in which:

A and $A_1$, independently of each other, are residues of formula:

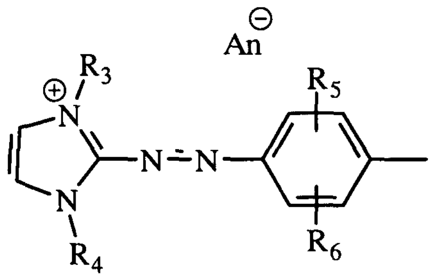

Z represents an aliphatic or aromatic diamine residue,
$R_1$ and $R_2$, independently of each other, represent a hydrogen atom, a $C_1$-$C_4$ alkyl group or may form, together with two nitrogen atoms to which they are attached or with Z and $Z_2$, a 5-, 6- or 7-membered ring,
X represents the- residue of a chain unit forming a bridge,
n represents an integer 2, 3 or 4,
$Z_1$ represents a residue of aromatic diamines,
$Z_2$ represents a residue of aliphatic diamines,
KK represents a residue of a coupling compound,
$R_3$ and $R_4$, independently of each other, represent a hydrogen atom or a $C_1$-$C_4$ alkyl group,
$R_5$ and $R_6$, independently of each other, represent a hydrogen atom, a $C_1$-$C_4$ alkyl group or a $C_1$-$C_4$ alkoxy group,
An represents a colourless anion.

7. Composition according to any one of Claims 1 to 6, **characterized in that** the cationic direct dye is chosen from Basic Red 51, Basic Yellow 87 and Basic Orange 31.

**8.** Composition according to any one of the preceding claims, **characterized in that** the concentration of cationic direct dye is between 0.001% and 20%, preferably between 0.01% and 10% and more preferably from 0.1% to 5% of the total weight of the composition.

**9.** Composition according to any one of the preceding claims, **characterized in that** the peroxygenated salt is chosen from alkali metal or alkaline-earth metal persulphates, perborates, percarbonates and peroxide.

**10.** Composition according to Claim 9, **characterized in that** the peroxygenated salt is sodium persulphate or potassium persulphate.

**11.** Composition according to any one of the preceding claims, **characterized in that** the concentration of the peroxygenated salts is between 10% and 70% and preferably between 20% and 60% of the total weight of the composition.

**12.** Composition according to any one of the preceding claims, **characterized in that** the alkaline agent is chosen from urea, ammonium chloride, ammonium sulphate, ammonium phosphate or ammonium nitrate, and alkali metal or alkaline-earth metal silicates, phosphates or carbonates.

**13.** Composition according to any one of the preceding claims, **characterized in that** the concentration of alkaline agent is between 0.01% and 40% and preferably between 0.1% and 30% of the total weight of the composition.

**14.** Composition according to any one of the preceding claims, **characterized in that** the inert organic liquid(s) is (are) chosen from the group formed by polydecenes of formula $C_{10n}H_{[(20n)+2]}$ in which n ranges from 3 to 9 and preferably from 3 to 7, and esters of fatty alcohols or of fatty acids.

**15.** Composition according to any one of the preceding claims, **characterized in that** it comprises at least one nonionic and/or anionic amphiphilic polymer comprising at least one fatty chain.

**16.** Composition according to any one of the preceding claims, **characterized in that** the concentration of nonionic and/or anionic amphiphilic polymer comprising at least one fatty chain is between 0.01% and 30% and preferably between 0.01% and 15% of the total weight of the composition.

**17.** Composition according to any one of the preceding claims, **characterized in that** it comprises beeswax as adjuvant.

**18.** Ready-to-use composition for simultaneously bleaching and dyeing human keratin fibres, in particular the hair, **characterized in that** it results from extemporaneously mixing a pasty anhydrous composition as defined according to any one of Claims 1 to 17 and a composition comprising hydrogen peroxide.

**19.** Ready-to-use composition according to Claim 18, **characterized in that** the said pasty anhydrous composition comprises a mixture of an anhydrous bleaching composition A in paste form containing at least one peroxygenated salt, at least one alkaline agent and at least one inert organic liquid, and a composition B containing at least one cationic direct dye.

**20.** Process for simultaneously bleaching and dyeing the keratin fibres, in particular the hair, comprising the steps consisting in:

- mixing, before use, an anhydrous bleaching and dyeing composition in paste form defined according to any one of Claims 1 to 17 with an aqueous hydrogen peroxide composition.
- applying the mixture obtained to the area of fibres to be treated,
- leaving the mixture on the fibres for a time generally of between 3 and 60 minutes and preferably between 5 and 40 minutes.
- removing the mixture by rinsing with water, followed by washing with a shampoo and then optionally drying.

**21.** Two-compartment device, or kit, for bleaching and dyeing human keratin fibres, and more particularly the hair, **characterized in that** the first compartment contains a pasty anhydrous composition as described in any one of Claims 1 to 17, and the second compartment contains an aqueous hydrogen peroxide composition.

**22.** Multi-compartment device or kit for bleaching and dyeing human keratin fibres, and more particularly the hair, **characterized in that** the first compartment contains a pasty anhydrous bleaching composition A which can contain

up to 1% by weight of water and contains at least one peroxygenated salt, at least one alkaline agent and at least one inert organic liquid, the second compartment contains a composition B containing at least one cationic direct dye, and the third compartment contains an aqueous hydrogen peroxide solution.

## Patentansprüche

1. Zusammensetzung, die pastös und wasserfrei ist und bis zu 1 Gew.-% Wasser enthalten kann, zum Entfärben und gleichzeitigen Färben von menschlichen Keratinfasern, insbesondere Haaren, die in einem zum Färben geeigneten Medium enthält:

    - mindestens ein Peroxysalz,
    - mindestens einen alkalischen Stoff,
    - bezogen auf das Gesamtgewicht der Zusammensetzung 15 bis 35 Gew.-% mindestens einer inerten organischen Flüssigkeit, die unter den Polydecenen der Formel $C_{10n}H_{[(20n)+2]}$, worin n im Bereich von 3 bis 9 liegt, Estern von Fettalkoholen oder Fettsäuren, Zuckerestern oder Zuckerdiestern von $C_{12-24}$-Fettsäuren, cyclischen Ethern oder cyclischen Estern, Siliconölen, Mineralölen oder pflanzlichen Ölen ausgewählt ist, und
    - mindestens einen kationischen Direktfarbstoff.

2. Zusammensetzung nach Anspruch 1, **dadurch gekennzeichnet, dass** der kationische Direktfarbstoff unter den Xanthen-Farbstoffen, Azofarbstoffen, Azomethin-Farbstoffen und Methin-Farbstoffen ausgewählt ist.

3. Zusammensetzung nach Anspruch 1 oder 2, **dadurch gekennzeichnet, dass** der kationische Direktfarbstoff ein heterocyclischer Direktfarbstoff ist.

4. Zusammensetzung nach Anspruch 3, **dadurch gekennzeichnet, dass** der Direktfarbstoff mindestens eine kationische Ladung an einem Heterocyclus aufweist.

5. Zusammensetzung nach Anspruch 4, **dadurch gekennzeichnet, dass** der Direktfarbstoff ein direktziehender Azofarbstoff, Azomethin-Farbstoff oder Methin-Farbstoff ist, der mindestens eine kationische Ladung an einem Heterocyclus aufweist.

6. Zusammensetzung nach einem der Ansprüche 2 bis 5, **dadurch gekennzeichnet, dass** der kationische Direktfarbstoff unter den folgenden Farbstoffen ausgewählt ist: Acid Red 52, Basic Blue 41, Basic Blue 67, Basic Brown 1, Basic Brown 4, Basic Red 18, Basic Red 22, Basic Red 46, Basic Red 104, Basic Violet 35, Basic Yellow 45, Basic Yellow 57, Basic Yellow 67, Basic Red 14, Basic Yellow 13 und Basic Yellow 29 sowie den folgenden Farbstoffen:

    - Farbstoffen der Formel (I):

    $$G\text{-}N\text{=}N\text{-}J \qquad (I)$$

    worin bedeuten:

    das Symbol G eine Gruppe, die unter den folgenden Strukturen $G_1$ bis $G_3$ ausgewählt ist:

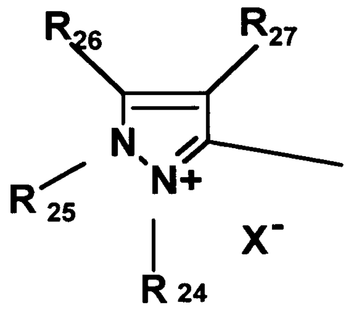

$G_1$

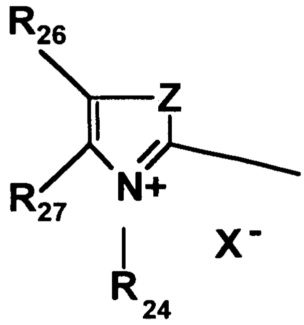

$G_2$

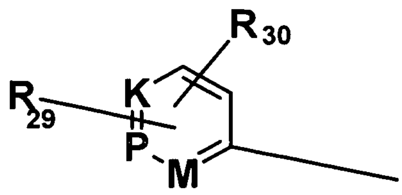

$G_3$

worin bedeuten:

$R_{24}$ eine $C_{1-4}$-Alkylgruppe, eine Phenylgruppe, die mit einer $C_{1-4}$-Alkylgruppe oder einem Halogenatom substituiert sein kann, das unter Chlor, Brom, Iod und Fluor ausgewählt ist;
$R_{25}$ eine $C_{1-4}$-Alkylgruppe oder eine Phenylgruppe;
$R_{26}$ und $R_{27}$, die gleich oder verschieden sind, $C_{1-4}$-Alkyl, Phenyl, oder sie bilden in $G_1$ gemeinsam einen Benzolring, der mit einer oder mehreren $C_{1-4}$-Alkylgruppen, $C_{1-4}$-Alkoxygruppen oder $NO_2$ substituiert ist, oder sie bilden in $G_2$ gemeinsam einen Benzolring, der gegebenenfalls mit einer oder mehreren $C_{1-4}$-Alkylgruppen, $C_{1-4}$-Alkoxygruppen oder $NO_2$ substituiert ist;
$R_{26}$ kann ferner ein Wasserstoffatom bedeuten;
Z ein Sauerstoffatom, ein Schwefelatom oder eine Gruppe $-NR_{25}$; M eine Gruppe -CH, -CR (wobei R $C_{1-4}$-Alkyl bedeutet) oder $-NR_{28}(X^-)_r$;
K eine Gruppe -CH, -CR (wobei R $C_{1-4}$-Alkyl bedeutet) oder $-NR_{28}(X^-)_r$;
P eine Gruppe -CH, -CR (wobei R $C_{1-4}$-Alkyl bedeutet) oder $-NR_{28}(X^-)_r$;
r Null oder 1;
$R_{28}$ ein Atom O-, $C_{1-4}$-Alkoxy oder $C_{1-4}$-Alkyl;
$R_{29}$ und $R_{30}$, die gleich oder verschieden sind, ein Wasserstoffatom oder ein Halogenatom, das unter Chlor, Brom, Iod und Fluor ausgewählt ist, $C_{1-4}$-Alkyl, $C_{1-4}$-Alkoxy oder $-NO_2$;
X- ein Anion, das vorzugsweise unter Chlorid, Iodid, Methylsulfat, Ethylsulfat, Acetat und Perchlorat ausgewählt ist;

das Symbol J:

- (a) eine Gruppe der folgenden Struktur $J_1$:

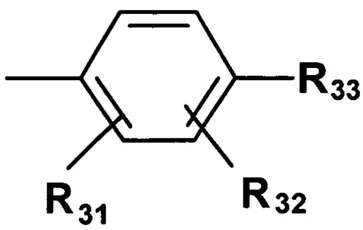

$J_1$

worin bedeuten:

$R_{31}$ ein Wasserstoffatom, ein Halogenatom, das unter Chlor, Brom, Iod und Fluor ausgewählt ist, $C_{1-4}$-Alkyl, $C_{1-4}$-Alkoxy, eine Gruppe -OH, $-NO_2$, $-NHR_{34}$, $-NR_{35}R_{36}$, -NHCO-alkyl($C_{1-4}$), oder $R_{31}$ bildet mit $R_{32}$ einen 5- oder 6-gliedrigen Ring, der gegebenenfalls ein oder mehrere Heteroatome aufweist, die unter Stickstoff, Sauerstoff oder Schwefel ausgewählt sind;
$R_{32}$ ein Wasserstoffatom, ein Halogenatom, das unter Chlor, Brom, Iod und Fluor ausgewählt ist, $C_{1-4}$-Alkyl, $C_{1-4}$-Alkoxy, oder $R_{32}$ bildet mit $R_{33}$ oder $R_{34}$ einen 5- oder 6-gliedrigen Ring, der gegebenenfalls ein oder mehrere Heteroatome enthält, die unter Stickstoff, Sauerstoff oder Schwefel ausgewählt sind;
$R_{33}$ ein Wasserstoffatom, eine Gruppe -OH, eine Gruppe $-NHR_{34}$, eine Gruppe $-NR_{35}R_{36}$;
$R_{34}$ ein Wasserstoffatom, $C_{1-4}$-Alkyl, $C_{1-4}$-Monohydroxyalkyl, $C_{2-4}$-Polyhydroxyalkyl, Phenyl;
$R_{35}$ und $R_{36}$, die gleich oder verschieden sind, $C_{1-4}$-Alkyl, $C_{1-4}$-Monohydroxyalkyl, $C_{2-4}$-Polyhydroxyalkyl;

- (b) eine stickstoffhaltige, 5- oder 6-gliedrige heterocyclische Gruppe, die weitere Heteroatome und/oder carbonylhaltige Gruppen enthalten kann und mit einer oder mehreren $C_{1-4}$-Alkylgruppen, Aminogruppen oder Phenylgruppen substituiert sein kann,
und insbesondere eine Gruppe der folgenden Struktur $J_2$:

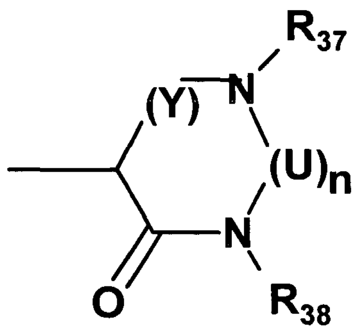

$J_2$

worin bedeuten:

$R_{37}$ und $R_{38}$, die gleich oder verschieden sind, ein Wasserstoffatom, $C_{3-10}$-Alkyl, Phenyl;
Y die Gruppe -CO- oder die Gruppe $-C(CH_3)=$;
n 0 oder 1, wobei, wenn n 1 ist, U die Gruppe -CO- bedeutet;

- Verbindungen der folgenden Formel (II):

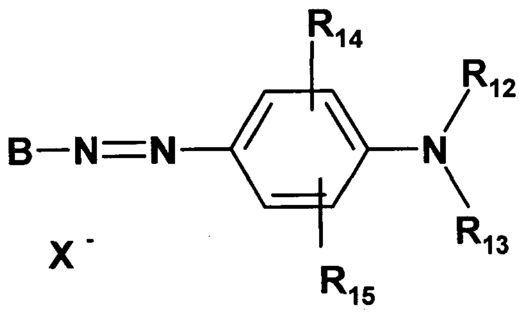

(II)

worin bedeuten:

$R_{12}$ ein Wasserstoffatom oder $C_{1-4}$-Alkyl,
$R_{13}$ ein Wasserstoffatom, eine Alkylgruppe, die mit einer Gruppe -CN oder einer Aminogruppe substituiert sein kann, 4'-Aminophenyl, oder $R_{13}$ bildet mit $R_{12}$ einen gegebenenfalls sauerstoffhaltigen und/oder stickstoffhaltigen Heterocyclus, der mit einer Gruppe $C_{1-4}$-Alkyl substituiert sein kann,
$R_{14}$ und $R_{15}$, die gleich oder verschieden sind, ein Wasserstoffatom, ein Halogenatom, wie Brom, Chlor, Iod oder Fluor, $C_{1-4}$-Alkyl oder $C_{1-4}$-Alkoxy, eine Gruppe -CN;
$X^-$ ein Anion, das vorzugsweise unter Chlorid, Methylsulfat
und Acetat ausgewählt ist;
B eine Gruppe, die unter den folgenden Strukturen B 1 bis B6 ausgewählt ist:

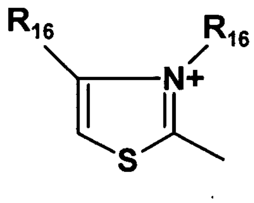

B1

;

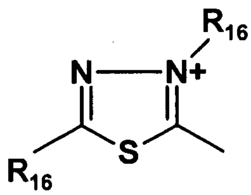

B2

;

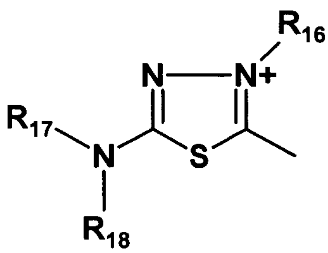

B3

;

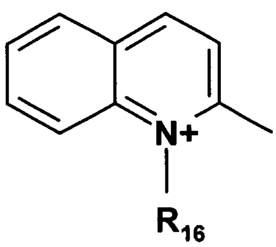

B4

;

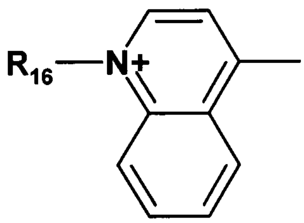

B5

und

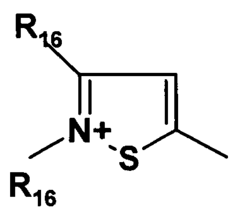

B6

;

worin $R_{16}$ $C_{1-4}$-Alkyl bedeutet und $R_{17}$ und $R_{18}$, die gleich oder verschieden sind, ein Wasserstoffatom oder $C_{1-4}$-Alkyl bedeuten;

- Verbindungen der folgenden Formeln (III) und (IV):

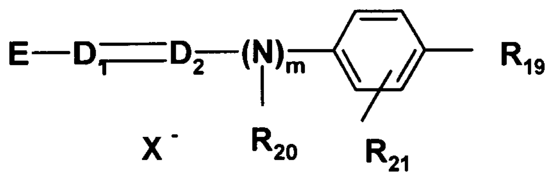

(III)

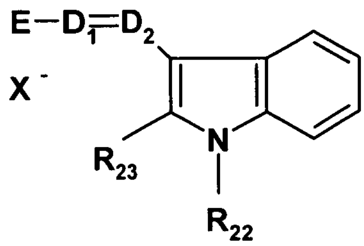

(IV)

worin bedeuten:

- $R_{19}$ ein Wasserstoffatom, $C_{1-4}$-Alkoxy, ein Halogenatom, wie Brom, Chlor, Iod oder Fluor, oder eine Aminogruppe,
- $R_{20}$ ein Wasserstoffatom, $C_{1-4}$-Alkyl, oder $R_{20}$ bildet mit einem Kohlenstoffatom des Benzolrings einen gegebenenfalls sauerstoffhaltigen und/oder mit einer oder mehreren $C_{1-4}$-Alkylgruppen substituierten Heterocyclus,
- $R_{21}$ ein Wasserstoffatom oder ein Halogenatom, wie Brom, Chlor, Iod oder Fluor,
- $R_{22}$ und $R_{23}$, die gleich oder verschieden sind, ein Wasserstoffatom oder $C_{1-4}$-Alkyl,
- $D_1$ und $D_2$, die gleich oder verschieden sind, ein Stickstoffatom oder die Gruppe -CH,
- m 0 oder 1,
- mit der Maßgabe, dass $D_1$ und $D_2$ gleichzeitig eine Gruppe -CH bedeuten und m = 0, wenn $R_{19}$ eine unsubstituierte Aminogruppe bedeutet,
- $X^-$ ein Anion, das vorzugsweise unter Chlorid, Methylsulfat und Acetat ausgewählt ist,
- E eine Gruppe, die unter den folgenden Strukturen E1 bis E8 ausgewählt ist:

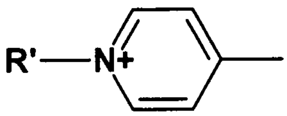

;

E1

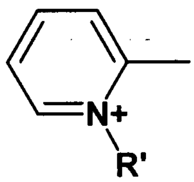

;

E2

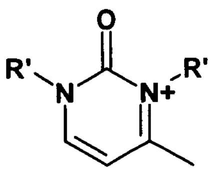

E3

;

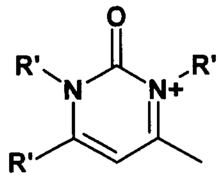

E4

;

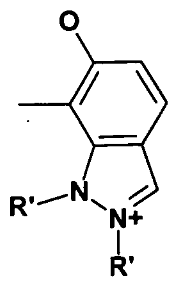

E5

;

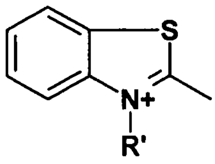

E6

;

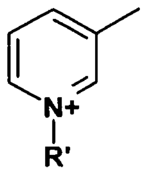

E7

und

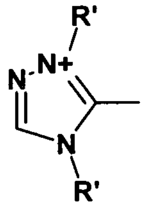

E8

worin R' $C_{1-4}$-Alkyl bedeutet;
wenn m 0 bedeutet und $D_1$ ein Stickstoffatom ist, kann E auch eine Gruppe der folgenden Struktur E9 sein:

E9

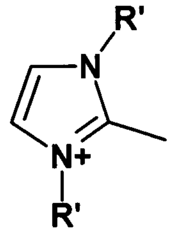

worin R' eine $C_{1-4}$-Alkylgruppe bedeutet;

- Farbstoffen der Formel (V);

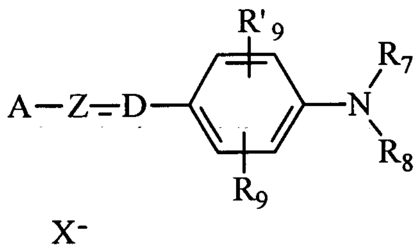

worin bedeuten:

Z und D, die gleich oder verschieden sind, ein Stickstoffatom oder die Gruppe -CH-,
$R_7$ und $R_8$, die gleich oder verschieden sind, ein Wasserstoffatom; eine $C_{1-4}$-Alkylgruppe, die mit einer Gruppe -CN, -OH oder $-NH_2$ substituiert sein kann, oder sie bilden mit einem Kohlenstoffatom des Benzolrings einen gegebenenfalls sauerstoffhaltigen oder stickstoffhaltigen Heterocyclus, der mit einer oder mehreren $C_{1-4}$-Alkylgruppen substituiert sein kann; 4'-Aminophenyl,
$R_9$ und $R'_9$, die gleich oder verschieden sind, ein Wasserstoffatom, ein Halogenatom, das unter Chlor, Brom, Iod und Fluor ausgewählt ist, Cyano, $C_{1-4}$-Alkyl, $C_{1-4}$-Alkoxy oder Acetyloxy,
$X^-$ ein Anion, das vorzugsweise unter Chlorid, Methylsulfat und Acetat ausgewählt ist,
A eine Gruppe, die unter den folgenden Strukturen A1 bis A 19 ausgewählt ist:

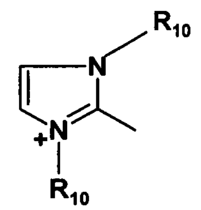

$A_1$ ;

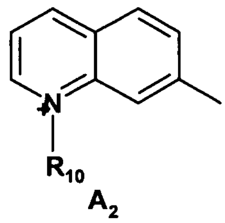

$A_2$ ;

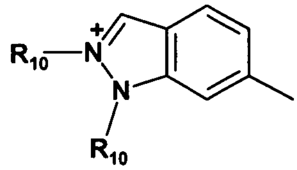

$A_3$ ;

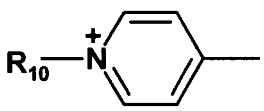

$A_4$ ;

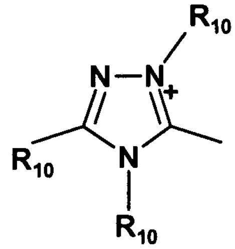

$A_5$ ;

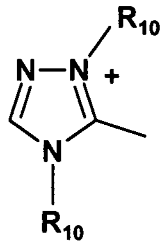

$A_6$ ;

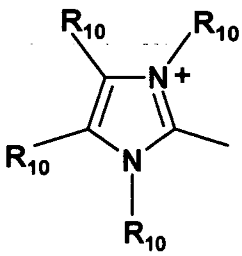

$A_7$ ;

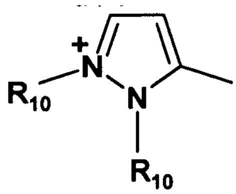

$A_8$ ;

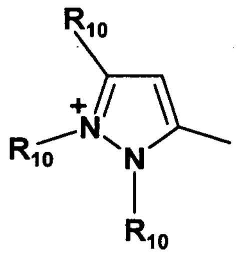

$A_9$ ;

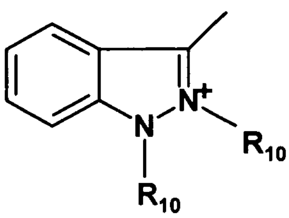

$A_{10}$ ;

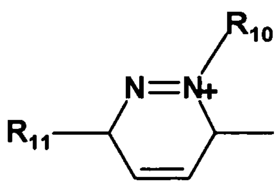

$A_{11}$ ;

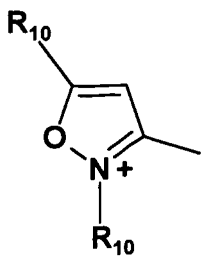

$A_{12}$ ;

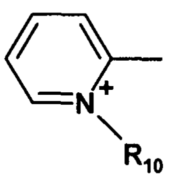

$A_{13}$ ;

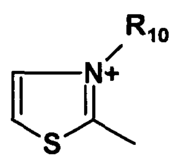

$A_{14}$ ;

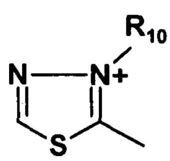

$A_{15}$ ;

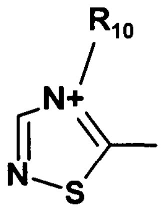

**$A_{16}$**

;

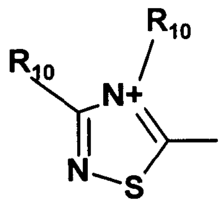

**$A_{17}$**

;

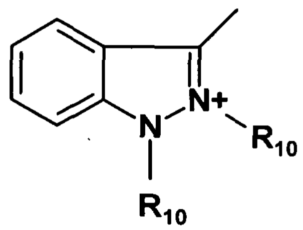

**$A_{18}$**

und

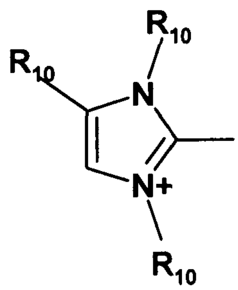

**$A_{19}$**

worin $R_{10}$ eine $C_{1-4}$-Alkylgruppe bedeutet, die mit einer Hydroxygruppe substituiert sein kann, und $R_{11}$ eine $C_{1-4}$-Alkoxygruppe bedeutet,

- Verbindungen der Formeln (VI), (VII) und (VIII):

**(VI)** 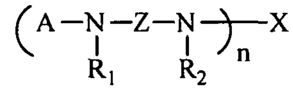

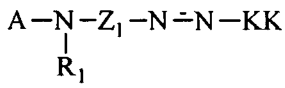 **(VII)**

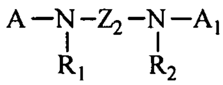 **(VIII)**

worin bedeuten:

A und $A_1$ unabhängig voneinander Reste der folgenden Formel:

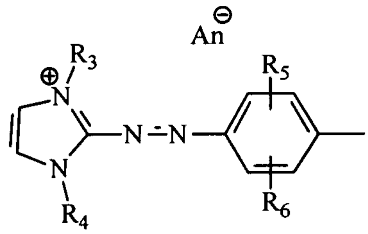

Z ein Rest einer aliphatischen oder aromatischen Diamingruppe,
$R_1$ und $R_2$ unabhängig voneinander ein Wasserstoffatom, $C_{1-4}$-Alkyl, oder sie können gemeinsam mit zwei Stickstoffatomen, an die sie gebunden sind, oder mit Z und $Z_2$ einen 5-, 6- oder 7-gliedrigen Ring bilden,
X den Rest einer eine Brücke bildenden Verknüpfung,
n eine ganze Zahl 2, 3 oder 4,
$Z_1$ einen aromatischen Diaminrest,
$Z_2$ einen aliphatischen Diaminrest,
KK eine Kupplergruppe,
$R_3$ und $R_4$ unabhängig voneinander ein Wasserstoffatom oder $C_{1-4}$-Alkyl,
$R_5$ und $R_6$ unabhängig voneinander ein Wasserstoffatom, $C_{1-4}$-Alkyl oder $C_{1-4}$-Alkoxy,
An ein farbloses Anion.

**7.** Zusammensetzung nach einem der Ansprüche 1 bis 6, **dadurch gekennzeichnet, dass** der kationische Direktfarbstoff unter Basic Red 51, Basic Yellow 87 und Basic Orange 31 ausgewählt ist.

**8.** Zusammensetzung nach einem der vorhergehenden Ansprüche, **dadurch gekennzeichnet, dass** die Konzentration des kationischen Direktfarbstoffes im Bereich von 0,001 bis 20 %, vorzugsweise 0,01 bis 10 % und noch bevorzugter 0,1 bis 5 % des Gesamtgewichts der Zusammensetzung liegt.

**9.** Zusammensetzung nach einem der vorhergehenden Ansprüche, **dadurch gekennzeichnet, dass** das Peroxysalz unter den Persulfaten, Perboraten, Percarbonaten und Peroxiden von Alkalimetallen oder Erdalkalimetallen ausgewählt ist.

**10.** Zusammensetzung nach Anspruch 9, **dadurch gekennzeichnet, dass** es sich bei dem Peroxysalz um Natriumpersulfat oder Kaliumpersulfat handelt.

**11.** Zusammensetzung nach einem der vorhergehenden Ansprüche, **dadurch gekennzeichnet, dass** die Konzentration der Peroxysalze im Bereich von 10 bis 70 % und vorzugsweise 20 bis 60 % des Gesamtgewichts der Zusammensetzung liegt.

**12.** Zusammensetzung nach einem der vorhergehenden Ansprüche, **dadurch gekennzeichnet, dass** der alkalische Stoff unter Harnstoff, Ammoniumchlorid, Ammoniumsulfat, Ammoniumphosphat oder Ammoniumnitrat, Silicaten, Phosphaten oder Carbonaten von Alkalimetallen oder Erdalkalimetallen ausgewählt ist.

**13.** Zusammensetzung nach einem der vorhergehenden Ansprüche, **dadurch gekennzeichnet, dass** die Konzentration des alkalischen Stoffes im Bereich von 0,01 bis 40 % und vorzugsweise 0,1 bis 30 % des Gesamtgewichts der Zusammensetzung liegt.

**14.** Zusammensetzung nach einem der vorhergehenden Ansprüche, **dadurch gekennzeichnet, dass** die organische (n) inerte(n) Flüssigkeit(en) unter den Polydecenen der Formel $C_{10n}H_{[(20n)+2]}$, worin n im Bereich von 3 bis 9 und vorzugsweise 3 bis 7 liegt, und Estern von Fettalkoholen oder Fetts äuren ausgewählt ist.

**15.** Zusammensetzung nach einem der vorhergehenden Ansprüche, **dadurch gekennzeichnet, dass** sie mindestens ein nichtionisches und/oder anionisches amphiphiles Polymer enthält, das mindestens eine Fettkette aufweist.

**16.** Zusammensetzung nach einem der vorhergehenden Ansprüche, **dadurch gekennzeichnet, dass** die Konzentration des nichtionischen und/ oder anionischen amphiphilen Polymers, das mindestens eine Fettkette aufweist, im

Bereich von 0,01 bis 30 % und vorzugsweise 0,01 bis 15 % des Gesamtgewichts der Zusammensetzung liegt.

**17.** Zusammensetzung nach einem der vorhergehenden Ansprüche, **dadurch gekennzeichnet, dass** sie als Adjuvans Bienenwachs enthält.

**18.** Gebrauchsfertige Zusammensetzung zum Entfärben und gleichzeitigen Färben von menschlichen Keratinfasern und insbesondere Haaren, **dadurch gekennzeichnet, dass** sie durch bedarfsgemäßes Mischen einer pastösen wasserfreien Zusammensetzung, wie sie in einem der Ansprüche 1 bis 17 definiert ist, und einer Zusammensetzung, die Wasserstoffperoxid enthält, gebildet wird.

**19.** Gebrauchsfertige Zusammensetzung nach Anspruch 18, **dadurch gekennzeichnet, dass** die pastöse wasserfreie Zusammensetzung ein Gemisch aus einer wasserfreien Zusammensetzung A zum Entfärben in Pastenform, die mindestens ein Peroxysalz, mindestens einen alkalischen Stoff und mindestens eine organ i-sche inerte Flüssigkeit enthält, und einer Zusammensetzung B, die mindestens einen kationischen Direktfarbstoff enthält, enthält.

**20.** Verfahren zum Entfärben und gleichzeitigen Färben von menschlichen Keratinfasern und insbesondere Haaren, das die folgenden Schritte umfasst:

- Mischen einer wasserfreien Zusammensetzung zum Entfärben und Färben in Pastenform nach einem der Ansprüche 1 bis 17 vor der Anwendung mit einer wässrigen Wasserstoffperoxidzusammensetzung,
- Aufbringen des erhaltenen Gemisches auf den zu behandel n-den Faserbereich,
- Einwirkenlassen während einer Zeitspanne im Allgemeinen von 3 bis 60 Minuten und vorzugsweise 5 bis 40 Minuten,
- Entfernen des Gemisches durch Spülen mit Wasser und anschließende Wäsche mit einem Haarwaschmittel, worauf gegebenenfalls getrocknet wird.

**21.** Vorrichtung mit zwei Abteilungen, oder Kit, zum Entfärben und Färben von menschlichen Keratinfasern und insbesondere Haaren, **dadurch gekennzeichnet, dass** die erste Abteilung eine wasserfreie pastöse Zusammensetzung, wie sie in einem der Ansprüche 1 bis 17 definiert ist, und die zweite Abteilung eine wässrige Wasserstoffperoxidzusammensetzung enthält.

**22.** Vorrichtung mit mehreren Abteilungen, oder Kit, zum Entfärben und Färben von menschlichen Keratinfasern und insbesondere Haaren, **dadurch gekennzeichnet, dass** die erste Abteilung eine wasserfreie und pastöse Zusammensetzung A zum Entfärben, die bis zu 1 Gew.-% Wasser enthalten kann und mindestens ein Peroxysalz, mindestens einen alkalischen Stoff und mindestens eine organische inerte Flüssigkeit enthält, die zweite Abteilung eine Zusammensetzung B, die mindestens einen kationischen Direktfarbstoff enthält, und die dritte Abteilung eine wässrige Wasserstoffperoxidzusammensetzung enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 19721785
- DE 19721797
- DE 3814685
- DE 3814356 A1
- DE 19723538 C1
- EP 1025834 A
- EP 714954 A
- WO 9501772 A
- WO 9515144 A
- EP 1170000 A
- EP 1166753 A
- EP 1166754 A
- EP 1170001 A
- FR 2788974
- FR 2788976
- US 3915921 A
- US 4509949 A
- EP 0216479 B2
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- FR 2080759
- FR 2190406
- FR 2320330
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- US 4157388 A
- US 4390689 A
- US 4702906 A
- US 4719282 A
- FR 2137684
- US 3879376 A
- FR 1400366
- US 5221534 A
- US 4540510 A
- FR 2633940
- US 2528378 A
- US 2781354 A


### Littérature non-brevet citée dans la description


- Chemistry and Technology of Silicones. Academic Press, 1968
- **G. FONNUM ; J. BAKKE ; FK. HANSEN.** *Colloid Polym. Sci,* 1993, vol. 271, 380-389
- **ROBERT L. DAVIDSON.** Handbook of Water soluble gums and resins. Mc Graw Hill Book Company, 1980
- Handbook of Surfactants. Blackie & Son, 1991, 116-178